# EUROPÄISCHE PATENTSCHRIFT

(11) **EP 3 362 463 B1**
(45) Veröffentlichungstag und Bekanntmachung des Hinweises auf die Patenterteilung: **29.04.2020**
(21) Anmeldenummer: 16781426.8
(22) Anmeldetag: 13.10.2016
(51) Int. Cl.: C07K 1/04

(54) **ULTRA-HOCHDICHTE OLIGOMERARRAYS UND VERFAHREN ZU DEREN HERSTELLUNG**
ULTRAHIGH-DENSITY OLIGOMER ARRAYS AND METHOD OF PRODUCTION THEREOF
MATRICES OLIGOMÈRES À ULTRAHAUTE DENSITÉ ET PROCÉDÉ POUR LEUR FABRICATION

(30) Priorität: 15.10.2015 DE 102015117567
(43) Veröffentlichungstag der Anmeldung: 22.08.2018
(73) Patentinhaber: Karlsruher Institut für Technologie, 76131 Karlsruhe (DE)
(72) Erfinder: NESTEROV-MUELLER, Alexander, 76661 Phillipsburg (DE); BYKOVSKAYA, Valentina, 396070 Novovoronezh Voronezh region (RU); VON BOJNICIC-KNINSKI, Clemens Matthias, 76228 Karlsruhe (DE); LOEFFLER, Felix Friedrich, 69115 Heidelberg (DE); POPOV, Roman, 76131 Karlsruhe (DE); RIDDER, Barbara, 76698 Ubstadt-Weiher (DE); BREITLING, Frank, 69115 Heidelberg (DE); MATTES, Daniela Silke, 76351 Linkenheim (DE)
(74) Vertreter: Witte, Weller & Partner Patentanwälte mbB
(86) Internationale Anmeldenummer: PCT/EP2016/074542
(87) Internationale Veröffentlichungsnummer: WO 2017/064165

(56) Entgegenhaltungen:
- WO-A1-2014/169928
- US-B1- 6 319 668
- ALEXANDER NESTEROV-MUELLER ET AL: "Particle-Based Microarrays of Oligonucleotides and Oligopeptides", MICROARRAYS, Bd. 3, Nr. 4, 28. Oktober 2014 (2014-10-28), Seiten 245-262, XP055330643, DOI: 10.3390/microarrays3040245

## Beschreibung

Die vorliegende Erfindung betrifft einen Oligomerarray und ein Verfahren zur Herstellung eines Oligomerarrays, wie beispielsweise eines Peptidarrays. Weiterhin wird die Verwendung eines derartigen Oligomerarrays zur Bestimmung eines Bindepartners beschrieben.

Oligomerarrays sind im Stand der Technik gut bekannt und betreffen die Gesamtheit der auf einem Träger bzw. Substrat kombinatorisch (aus einzelnen molekularen Bausteinen, wie bspw. Monomeren) synthetisierten Oligomere. Sie binden dabei kovalent in Form von Spots an einen Träger. Die Synthese dieser Oligomere beruht im Allgemeinen auf den chemischen Prinzipien der Festphasensynthese. Die Realisierung der parallelen Festphasensynthese, insbesondere der Transport der Monomere zu dem jeweiligen Syntheseort, ist dabei von entscheidender Bedeutung.

Derartige auf einem Oligomerarray immobilisierten Oligomere umfassen alle Arten von Oligomeren, die kombinatorisch aus mehreren Bausteinen synthetisiert werden können. Beispiele derartiger Bausteine umfassen Aminosäurederivate und Nukleotidderivate, wie Deoxyribonukleotidderivate und Ribonukleotidderivate.

Die vorstehend genannten Oligomerarrays sind insbesondere bei der Detektion/ Bestimmung von Bindepartnern von Interesse. Hierbei wird beispielsweise über Hybridisierung ein geeigneter Bindepartner ermittelt. Das Vorliegen eines entsprechenden Bindeereignisses kann beispielsweise die Inhibierung der Wirkung eines Biomoleküls begründen, wodurch die Verwendung des entsprechenden inhibierenden Oligomers als Medikament möglich wird. Die parallele Durchmusterung von möglichst vielen Oligomeren mit potenzieller biologischer Aktivität ist daher in der Medizin und angrenzenden Gebieten von hoher Bedeutung. Um die parallele Durchmusterung einer großen Anzahl von Oligomeren zu ermöglichen, ist insbesondere die Herstellung ultra-hochdichter Oligomerarrays von Interesse, bei denen mehr als 10⁶ Oligomere pro Quadratzentimeter bzw. Spots/ cm² auf dem Träger immobilisiert vorliegen.

Auch WO2014/169928 A1 enthüllt ein Verfahren zur Herstellung von Oligomerarrays.

Weitere Verfahren zur Herstellung von Oligonukleotiden umfassen Phage Display bzw. Ribosomal Display Verfahren. Hierbei werden synthetisch hergestellte Oligonukleotide mit dem Gen eines Phagenhüllproteins fusioniert, so dass nach erfolgter Transfektion eines Bakteriums jedes Bakterium eine andere Sorte eines Phagen "verpackt", der sich nur in der Sequenz der mit dem Phagenhüllprotein fusionierten Peptide unterscheidet (vgl. Smith, G.P. filamentous fusion phage - novel expression vectors that display cloned antigens on the virion surface; Science 228 (1985) pp. 1315-1317).

Bei der kombinatorischen Peptidsynthese mit Halbleiterchips handelt es sich beispielsweise um einen Hochspannungs-CMOS-Chip, dessen Oberfläche in verschiedene Elektroden unterteilt ist (M. Beyer et al., Science, 318 (5858), 1888, 2007). Durch die Programmierung des Chips können selektiv einzelne Elektroden eingeschaltet werden. Mittels der entstehenden elektrischen Felder werden geladene Partikel, welche als Träger der Monomere dienen, ortsgenau auf dessen Elektroden abgelagert. Die Synthese des Oligomerarrays kann entweder direkt auf der Chipoberfläche oder auf einem Zielträger (zum Beispiel einem Glasobjektträger) stattfinden. Hierfür wird das auf dem CMOS-Chip generierte Partikelmuster mit Hilfe eines elektrischen Feldes auf dessen Zielträger übertragen.

Weiterhin sind lithografische Verfahren bekannt, mit denen Spotdichten von bis zu 10⁶ Spots/cm² erzielt werden können (Fodor S.P.A. et al.; light-directed, spatially addressable parallel chemical synthesis. Science 251, pp. 767-773 (1991); und Legutki J.-B. Nat. Commun. 5,4785,2014). Mit Lichtmasken werden Schutzgruppen entsprechend des generierten Lichtmusters abgespalten, welche die Anbindung nachfolgender Aminosäureglieder ermöglicht. Neben der Notwendigkeit einer präzisen Positionierung der Syntheseträger hat ein derartiges lithografisches Verfahren den weiteren Nachteil, dass für jedes Monomer eine separate Kupplungsreaktion durchgeführt werden muss. Dies führt unweigerlich zu Nebenreaktionen, die verhindern, dass Peptidarrays mit ausreichend guter Qualität kommerziell erhältlich sind (Palloys J.P. et al.; individually addressable parallel peptide synthesis on microchips; Nature Biotechnology 20 (2002), pp. 922-926).

Im Falle des Combinatorial Laser Fusing (CLF) werden Monomer-enthaltende Partikel mit Hilfe von Laserstrahlung direkt auf einem Syntheseträger fixiert. Hierbei wird ein Laserstrahl über den Träger gelenkt, um die Partikel selektiv anzuschmelzen (Maerkle F. et al., High-Densitiy Peptide Arrays with Combinatorial Laser Fusing, Advanced Materials, Volume 26 (2014), pp. 3730-3734).

Ein weiteres Verfahren betrifft ein Xerographieverfahren, bei dem Tonerpartikel von einem 24-Farben-Laserdrucker verdruckt werden, die jeweils einen Aminosäurebaustein für die kombinatorische Synthese enthalten. Ein derartiges Verfahren wird beispielsweise in der WO 00/35940 beschrieben.

Das vorstehend genannte lithografische Verfahren benötigt eine Vielzahl von Kupplungszyklen, sofern damit ein Oligomerarray und insbesondere ein Peptidarray aufgebaut werden soll. Die Anzahl der Kupplungszyklen berechnet sich hierbei aus N x Y, wobei N die Anzahl der verschiedenen Monomere und Y die Länge der Oligomere bezeichnet. Wenn beispielsweise ein Array von 15mer Peptiden synthetisiert werden soll, in dem 20 Aminosäuren zum Einsatz gelangen, berechnet sich die Zahl der Kupplungszyklen zu 300. Aus diesem Grund werden lithografische Verfahren bisher nur erfolgreich für die Synthese von Oligonukleotidarrays eingesetzt. Infolge gibt es derzeit kein anderes kommerziell verfügbares Verfahren, mit dem in genügend hoher Qualität (Peptid-) Spotdichten von mehr als 40.000 Spots/cm² erzielt werden. Ein weiteres technisches Problem sind die komplizierten und aufwendigen Vorrichtungen, die für die lithografischen und auch für alle anderen Verfahren benötigt werden.

Die WO 2014/169928 A1 offenbart ein Verfahren zur kombinatorischen Partikelmanipulation zur Herstellung von hochdichten Molekülarrays sowie die daraus erhaltenen hochdichten Molekülarrays. Ein Verfahren zur Herstellung von hochdichten Molekülarrays mit einem Rastermaß von < 300 µm wird beschrieben wobei das Verfahren umfasst: (i) das Bereitstellen eines Zielträgers mit einer Vielzahl an diskreten Spots, (ii) das Konditionieren ausgewählter Spots durch selektive und direkte Einwirkung elektromagnetischer Strahlung, und (iii) das Umsetzen mindestens eines Monomers mit in den ausgewählten Spots des Zielträgers immobilisiert vorliegenden Reaktanden.

Vor diesem Hintergrund besteht daher die Aufgabe der vorliegenden Erfindung darin, ein Verfahren zur Verfügung zu stellen, mit dem Oligomerarrays mit wenigen Kupplungsschritten hergestellt werden können. Eine weitere Aufgabe besteht in der Bereitstellung eines Verfahrens mit dem einfach hochdichte und ultra-hochdichte Oligomerarrays hergestellt werden können. Eine weitere Aufgabe besteht in der Bereitstellung eines Verfahrens, bei dem Vorrichtungen zur Anwendung gelangen, die einen einfachen Aufbau aufweisen und kostengünstig in der Herstellung sind. Noch eine weitere Aufgabe besteht in der Bereitstellung eines Verfahrens, welches die schnelle Herstellung von Oligomerarrays ermöglicht, indem bspw. die exakte Positionierung des Substrats nicht benötigt wird. Noch eine weitere Aufgabe besteht in der Bereitstellung entsprechender Oligomerarrays und deren Verwendung bei der Bestimmung geeigneter Bindepartner.

Diese Aufgabe wird durch ein Verfahren zur Herstellung eines Oligomerarrays mit den folgenden Schritten gelöst:
a) Bereitstellen eines Substrats mit einer Vielzahl von Vertiefungen;
b) stochastisches Einbringen eines ersten Partikels mit einem ersten Molekül in eine Vertiefung derart, dass nicht im Vorfeld bestimmt wird, welches erste Molekül an welchem Ort fixiert wird;
c) Freisetzen des ersten Moleküls von dem ersten Partikel;
d) Binden des ersten Moleküls an ein zweites Molekül unter Bildung eines Oligomers, wobei das zweite Molekül in der Vertiefung immobilisiert ist, wobei nach dem Schritt (b), (c) oder (d) eine Bestimmung des detektierbaren Markers in Abhängigkeit einer Position der Vertiefung auf dem Oligomerarray erfolgt;
e) Wiederholen der Schritte (b) bis (d) unter Verlängerung des Oligomers; wobei mindestens ein erster Partikel und/oder ein erstes Moleküls einen detektierbaren Marker aufweist, wobei eine Vielzahl erster Partikel verwendet wird; und
(f) Erstellen einer 3D-Ablagerungsmaske aus den Bestimmungen des detektierbaren Markers in Abhängigkeit der Position der Vertiefung auf dem Oligomerarray, wobei die 3D-Ablagerungsmaske alle Informationen über den Ort und den Aufbau des Oligomers enthält.

Gemäß einem weiteren Aspekt der vorliegenden Erfindung wird die Verwendung eines Oligomerarrays zur Bestimmung eines Bindepartners bereitgestellt.

Die vorliegende Erfindung beruht auf dem Verzicht auf einen kontrollierten Transport der Monomere bzw. Moleküle zu den Syntheseorten. Stattdessen erfolgt der Transport der Monomere zu den Syntheseorten stochastisch bzw. zufällig, das heißt es kann nicht im Vorfeld bestimmt werden, welches Monomer an welchem Ort fixiert wird. Nach der Bindung der einzelnen Monomere wird lediglich deren Endposition registriert. Dadurch kann nach Vollendung des Arrays in der Rückschau eine 3D-Ablagerungsmaske erstellt werden, die alle notwendigen Informationen über den Ort und Aufbau der Oligomere eines jeden Spots enthält. Durch den Wegfall der kontrollierten Ablagerung der Moleküle wird dadurch die einfache und kostengünstige Produktion von ultra-hochdichten Oligomerarrays, und insbesondere Peptidarrays, mit Spotdichten von bis zu 1.000.000 Spots/cm² und mehr ermöglicht, da vorhandene Partikelgemische in die Vertiefungen eines Substrats, wie beispielsweise eines mikrostrukturierten Glasträgers, eingebracht, detektiert und anschließend zum Freisetzen und Binden der Monomere parallel verarbeitet, bspw. erhitzt, werden können.

Durch die Verwendung des Partikel-basierenden Transfers der Moleküle in eine Vertiefung kann (i) ein vereinfachter Transfer von Molekülen in eine Vertiefung des Substrats, beispielsweise durch Ablagerung oder Einreiben einer die Partikel enthaltenden Lösung in die Vertiefungen, bewerkstelligt werden. Weiterhin (ii) kann lediglich ein Oligomer aus (einem) Partikel in einer Vertiefung immobilisiert werden. Durch die Verwendung einer Partikelform, die zu der Form der Vertiefung passt, dass heißt für gewöhnlich weist das Partikel und die Vertiefung einen passenden Querschnitt auf, kann sichergestellt werden, dass (iii) ein Oligomer (aus einem Partikel mit einem bestimmten Querschnitt) in einer bestimmten Vertiefung mit dem passenden Querschnitt immobilisiert wird.

Die Zahl und Art der Partikel pro Vertiefung kann hierbei über den Querschnitt/Form der Vertiefung und Querschnitt/Form des Partikels gesteuert werden. Vorzugsweise, sind die Partikelabmessungen und der Querschnitt der Vertiefung derart gewählt, dass ein einzelnes Partikel in eine Vertiefung passt. Mehr bevorzugt ist der Querschnitt der Vertiefung im Wesentlichen kreisförmig und der Querschnitt des Partikels im Wesentlichen kreisförmig mit derart geringeren Abmessungen, dass ein einzelnes Partikel in eine Vertiefung passt. Die Tiefe der Vertiefung ist hierbei so gewählt, dass lediglich ein einzelnes Partikel in eine Vertiefung passt. Der vorliegende Ansatz ermöglicht (iv) ferner das Belegen bzw. Blockieren von bestimmten Vertiefungen, indem in derartige Vertiefungen Partikel eingebracht werden, die nicht im Rahmen der Oligomersynthese abgebaut werden, das heißt beispielsweise Partikel einer anderen chemischen Zusammensetzung als das Partikel, die zum Einbringen eines Bausteins, bspw. eines Monomers, in eine Vertiefung verwendet werden.

Gemäß dem erfindungsgemäßen Verfahren weist mindestens eines des ersten Partikels und/oder des ersten Moleküls einen detektierbaren Marker auf. Der detektierbare Marker kann beispielsweise ein Fluoreszenz- oder Lumineszenzmarker sein. Alternativ kann der Marker ein Polynukleotid sein, dessen Bestimmung mittels Hybridisierung erfolgt. Bei einer Vielzahl von verschiedenen (ersten) Molekülen, die in einem Syntheseschritt zur stochastischen Kupplung gebracht werden sollen, kann durch eine Wahl geeigneter detektierbarer Marker die Charakterisierung der entsprechend um eine Position verlängerten Oligomers erfolgen. Die Zahl der pro Kupplungsschritt erfolgten Anbindung verschiedener Oligomere ist hierbei nicht begrenzt, sondern erfordert lediglich eine entsprechende Kennzeichnung des ersten Moleküls und/oder des ersten Partikels. Es ist ferner klar, dass die Zahl der Markierungen nicht notwendigerweise mit der Anzahl der pro Reaktionsschritt eingebrachten ersten Partikel mit einem ersten Molekül übereinstimmt, sondern dass durch Kombination verschiedener Markierungen gleichfalls Mischfarben erzeugt werden können, die die Bestimmung bzw. Zuordnung des ersten Moleküls zu der entsprechenden Vertiefung unzweideutig ermöglichen.

In dem erfindungsgemäßen Verfahren kann nach einem der Schritte b), c) oder d) die Bestimmung des detektierbaren Marker erfolgen. Nach Schritt b) erfolgt hierbei vorzugsweise die Bestimmung eines detektierbaren Markers, der/ die auf der Partikeloberfläche vorliegt. Nach den Schritten c) und/ oder d) erfolgt die Bestimmung detektierbarer Marker, die an das erste Molekül kovalent gebunden sind.

Vorzugsweise weist das Verfahren zur Herstellung des Oligomerarrays die folgenden Schritte auf: a) Bereitstellen eines Substrats mit einer Vielzahl von Vertiefungen; b) stochastisches Einbringen eines ersten Partikels mit einem ersten Molekül in eine Vertiefung; c) Freisetzen des ersten Moleküls von dem ersten Partikel; d) Binden des ersten Moleküls an ein zweites Molekül unter Bildung eines Oligomers, wobei das zweite Molekül in der Vertiefung immobilisiert ist; e) optional Wiederholen der Schritte (b) bis (d) unter Verlängerung des Oligomers; wobei mindestens ein erster Partikels und/oder ein erstes Moleküls einen detektierbaren Marker aufweist. Eine Vielzahl erster Partikel wird verwendet. Vorzugsweise umfasst jedes der mehreren ersten Partikel jeweils identische erste Moleküle und jedes der mehreren ersten Partikel weist einen detektierbaren Marker auf, der von dem Marker eines anderen der mehreren ersten Partikel verschieden ist.

Vorzugsweise kommen die allgemein anerkannten Prinzipien der Festphasensysthese von beispielsweise der Synthese von Polynukleotiden, Polypeptiden und anderen Polymeren zum Einsatz. Die Durchführung derartiger Festphasensynthesen, einschließlich der Reagenzien, Reaktionsbedingungen und Schutzgruppenchemie etc., sind dem Fachmann bekannt und können ohne weiteres auf das vorliegende Verfahren übertragen werden. Dadurch wird es ermöglicht, Oligomerarrays für eine Vielzahl an verschiedenen Oligomerbausteinen bzw. Molekülen bereitzustellen. Es ist klar, dass die Oligomerarrays aus anderen Bausteinen als Nukleotiden bzw. Nukleotidderivaten oder Peptiden bzw. Peptidderivaten bestehen können. Polymere können auch verschiedene Bausteine gleichzeitig enthalten, beispielsweise Nukleotidderivate und Aminosäurederivate. Die Nukleotidderivate und Aminosäurederivate können hierbei natürlichen oder künstlichen Ursprungs sein. Im Prinzip kann ein Oligomer eine Abfolge beliebiger Moleküle umfassen.

Wie hierin verwendet betrifft der Begriff "Array" die flächige Anordnung einer Vielzahl von Oligomeren in Vertiefungen eines Substrats. Vorzugsweise handelt es sich um hochdichte Oligomerarrays mit einer Dichte von 10³ Spots/cm² oder mehr, vorzugsweise 10⁴ Spots/cm² oder mehr bzw. 10⁵ Spots/cm² oder mehr. Mehr bevorzugt handelt es sich um ultra-hochdichte Oligomerarrays mit einer Dichte von 10⁶ Spots/cm² oder mehr, 2,5 x 10⁷Spots/cm² oder mehr, 5 x 10⁷Spots/cm² oder mehr bzw. 7 x 10⁷Spots/cm² oder mehr.

Der Begriff "Oligomer" wie hierin verwendet betrifft (die maximale) Kettenlänge einer chemischen Verbindung aus einer Vielzahl von Molekülen. Die Moleküle können hierbei gleich oder verschieden sein. Vorzugsweise kommen gleichartige Moleküle, wie beispielsweise Aminosäuren bzw. Aminosäurederivate oder Nukleinsäuren bzw. Nukleinsäurederivate, zur Anwendung. Ein Oligomer ist somit durch dessen maximale Kettenlänge gekennzeichnet, die in der Regel 2 bis 30 Moleküleinheiten umfasst. Vorzugsweise umfasst ein Oligomer 2 bis 25 Moleküleinheiten, 3 bis 20 Moleküleinheiten, 4 bis 19 Moleküleinheiten, 5 bis 18 Moleküleinheiten, 6 bis 17 Moleküleinheiten, 8 bis 16 Moleküleinheiten, 9 bis 15 Moleküleinheiten, 10 bis 14 Moleküleinheiten, 11 bis 13 Moleküleinheiten oder 12 Moleküleinheiten. Mehr bevorzugt sind 5 bis 18 Moleküleinheiten, 8 bis 15 Moleküleinheiten und 12 bis 15 Moleküleinheiten. Der Begriff maximale Kettenlänge bezeichnet hierbei das Rückgrat des Oligomers, wobei ein Ende davon an dem Substrat entweder direkt oder über einen Spacer immobilisiert sein kann, und das andere Ende mit einem weiteren ersten Molekül versehen werden kann. Oligomere können auch Verzweigungen und/oder Ringschlüsse umfassen, wobei Seitenkettenfunktionalitäten der einzelnen Moleküle/Monomere mit einem geeigneten Reaktionspartner umgesetzt werden.

Der Begriff "Substrat", wie hier verwendet, betrifft den Träger eines Oligomerarrays und umfasst Materialien wie beispielsweise Glas, Quarz, Silizium, Keramik, Kunststoff, Metalle und/oder Kompositwerkstoffe. Bevorzugt sind Substrate aus Quarzglas, bspw. Objektträger aus Quarzglas.

Die Begriffe "Durchmesser" und "Abmessung", wie hier verwendet, betreffen den Durchmesser oder die Abmessung eines Partikels oder einer Vertiefung und liegen für gewöhnlich im Bereich einiger weniger µm, bspw. von 0,1 bis 100 µm. Der Durchmesser bzw. die Abmessungen des Partikels oder der Vertiefung werden durch das Herstellungsverfahren festgelegt. Bspw. kann die Strukturierung einer Oberfläche eines Substrats durch den Einsatz eines Lasers mit der gewünschten Genauigkeit erfolgen. Dem Fachmann ist jedoch klar, dass bei der vorliegenden Erfindung nicht die Genauigkeit des "Durchmessers" und der "Abmessung" entscheidend ist, sondern vielmehr das Passen eines Partikels in eine Vertiefung, vorzugsweise eines einzelnen Partikels in eine einzelne Vertiefung. Hierbei sind im Allgemeinen lediglich die Größenverhältnisse und Verhältnisse der Abmessungen entscheidend. Mithin können beispielsweise Partikel hergestellt werden die lediglich ungefähr einen zu einer Vertiefung passenden Durchmesser aufweisen, da größere Partikel ggf. entfernt, bspw. weggewaschen, werden können. Ferner kann die Belegung einer Vertiefung mit einem Partikel, vorzugsweise einer einzelnen Vertiefung mit einem einzelnen Partikel, durch die Verwendung des detektierbaren Markers verifiziert werden. Bspw. kann so festgestellt werden, ob vorzugsweise ein einzelnes Partikel in einer einzelnen Vertiefung vorliegt oder ob gegebenenfalls zwei oder mehrere Partikel in einer einzelnen Vertiefung vorliegen. Beispielsweise kann das Vorliegen zweier Partikel mit identischem detektierbarem (Fluoreszenz-) Marker in einer einzelnen Vertiefung gegenüber dem Vorliegen eines Partikels mit dem entsprechenden Marker in einer einzelnen Vertiefung durch die unterschiedliche (Fluoreszenz-) Intensität ermittelt werden. Sofern bspw. zwei Partikel mit verschiedenen detektierbarem (Fluoreszenz-) Marker in einer einzelnen Vertiefung vorliegen, ermöglichen die jeweiligen Marker, bspw. über verschiedene Absorptionsmaxima im Fluoreszenzspektrum, die eindeutige Bestimmung des Partikels. Der Durchmesser ist vorzugsweise der Mediandurchmesser.

Der Mediandurchmesser kann im Fall eines Partikels bspw. mittels Coulter-Zähler gemäß ISO 13319:2007 bestimmt werden. Im Rahmen der vorliegenden Erfindung wurde ein Beckman Coulter Multisizer 3 verwendet. Alle Messungen werden entsprechend der Herstellerangaben durchgeführt.

Die Abmessungen des Substrats können mittels Weißlichtinterferometrie gemäß EN ISO 25178 bestimmt werden. Im Rahmen der vorliegenden Erfindung wurde ein Bruker VSI Contour K0 verwendet. Alle Messungen werden entsprechend der Herstellerangaben durchgeführt. Geeignete Substrate können beispielsweise von der AMO GmbH, Aachen, Deutschland bezogen werden.

Der Begriff "Vertiefung" wie hierin verwendet betrifft eine Einbuchtung bzw. Senke bzw. Mulde in der Oberfläche eines Substrats. Die Vertiefungen sind hierbei flächig angeordnet und derart voneinander beabstandet, dass keine Überlappung erfolgt und somit einzelne, voneinander unterscheidbare Vertiefungen vorliegen. Die Zahl der Vertiefungen spiegelt hierbei die Dichte des Arrays wieder. Der Querschnitt der Vertiefungen und die Tiefe der Vertiefung ermöglicht die Aufnahme eines oder mehrerer Partikel. Vorzugsweise passt lediglich ein Partikel in eine Vertiefung. Eine Steuerung der Partikel pro Vertiefung kann hierbei durch die Wahl eines geeigneten Querschnitts der Vertiefung erfolgen, die zu dem Querschnitt des Partikels passt. Beispielsweise weist eine Vertiefung und ein erstes Partikel jeweils einen im Wesentlichen kreisförmigen oder vieleckigen Querschnitt, wie beispielsweise einen fünfeckigen, viereckigen oder dreieckigen Querschnitt, auf. Im Wesentlichen kreisförmig bzw. im Wesentlichen vieleckig beinhaltet hierbei, dass das Verhältnis der maximalen Länge der Vertiefung zur maximalen Breite der Vertiefung zwischen 1,0 und 1,2 liegt, vorzugsweise 1,0 bis 1,15, 1,0 bis 1,1, 1,0 bis 1,05 und 1,0 bis 1,01 beträgt. Vorzugsweise ist die Vertiefung ideal kreisförmig bzw. ideal vieleckig. Die Tiefe der Vertiefung ist vorzugsweise so gewählt, dass lediglich ein einzelnes Partikel in eine Vertiefung passt. Das Aspektverhältnis beträgt beispielsweise 0,5 bis 1,5, vorzugsweise 0,6 bis 1,4, 0,7 bis 1,3, 0,8 bis 1,2, 0,9 bis 1,1 und am meisten bevorzugt 1. Beispielhafte Durchmesser einer Vertiefung umfassen 0,1 bis 100 µm, vorzugsweise 0,8 bis 50 µm, 1 bis 15 µm, 2,5 bis 14 µm, 5 bis 13 µm, 6 bis 12 µm, 7 bis 11 µm oder 8 bis 10 µm, wie beispielsweise 9 µm. Der Mitte-zur-Mitte-Abstand (Pitch) der einzelnen Vertiefungen zueinander wird derartig gewählt, dass die Vertiefungen auf dem Substrat individualisiert vorliegen und ist ansonsten nicht weiter begrenzt. Der Pitch kann beispielsweise zwischen 1 und 70 µm, vorzugsweise 10 bis 40 µm, 20 bis 30 µm oder 25 µm betragen.

Die erfindungsgemäße Herstellung eines Oligomerarrays, wie beispielsweise eines Peptidarrays, besteht im Wesentlichen in der Abfolge folgender Verfahrensschritte:

Molekül enthaltende Partikel für die kombinatorische Synthese werden nach dem Zufallsprinzip in die Vertiefungen/Mikrokavitäten des Substrats gefüllt, wobei vorzugsweise ein Gemisch verschiedener Molekülpartikel verwendet wird, wie beispielsweise zwanzig verschiedene Arten von Partikeln, die jeweils eine der zwanzig unterschiedlichen, für die Festphasensynthese geeigneten Oligomere bzw. Oligomerderivate, wie beispielsweise Aminosäuren oder Aminosäurederivate, enthalten. Die Partikelgröße wird hierbei so gewählt, dass in jede Vertiefung lediglich ein Partikel passt. In einem Molekülpartikel können mehrere, voneinander verschiedene Moleküle in einem Partikel, wie beispielsweise 2 oder mehr voneinander verschiedene Moleküle, 3 oder mehr voneinander verschiedene Moleküle, 4 oder mehr voneinander verschiedene Moleküle, 5 oder mehr voneinander verschiedene Moleküle und 10 oder mehr voneinander verschiedene Moleküle vorliegen. Vorzugsweise liegen in einem Partikel Moleküle vor, die nicht voneinander verschieden sind, d.h. ein bestimmter Molekültyp. Hierbei ist auf die entsprechende Markierung der Partikel und/oder Moleküle zu achten, um eine eindeutige Identifizierung der Moleküle zu ermöglichen. Vorzugsweise sind die Moleküle im Inneren des Partikels enthalten, d.h. nicht kovalent an das Partikel gebunden.

Vorzugsweise handelt es sich bei den Molekülpartikeln um Monomerpartikel, in denen lediglich ein bestimmtes Molekül in/an einem Partikel vorliegt. Dadurch wird sichergestellt, dass lediglich ein bestimmtes Monomer in einer Vertiefung immobilisiert. Vorzugsweise ist das Molekül im Inneren des Partikels enthalten, d.h. nicht kovalent an das Partikel gebunden.

Beispielsweise kommen Partikel aus einem Styrol-Acrylat-Copolymer als Matrix mit einem Mediandurchmesser von 3 µm pro Partikel zur Verwendung. Weitere geeignete Partikel, deren Synthese, sowie deren Synthese mit darin befindlichen Monomeren können beispielsweise der WO 2014/169928 und DE 101 56 329 A1 entnommen werden. Das erste Molekül liegt hierbei vorzugsweise in dem ersten Partikel vor, so dass das erste Partikel das erste Molekül vollständig einschließt. Alternativ dazu kann das erste Molekül auch an der Oberfläche des ersten Partikels angebracht, bspw. kovalent daran gebunden, vorliegen.

In einem nächsten Schritt wird das erste Molekül von dem ersten Partikel bzw. aus dem ersten Partikel freigesetzt. Dies kann bei der Verwendung von Styrol-Acrylat-Copolymeren als Matrixmaterial des ersten Partikels durch Temperaturerhöhung erfolgen. Beispielsweise wird der Oligomerarray mit einer (Vielzahl) erster Partikel mit verschiedenen ersten Molekülen in eine Vielzahl von Vertiefungen eingebracht, so dass pro Vertiefung ein erstes Partikel vorliegt. Durch Erhitzen auf beispielsweise 80° C oder mehr, wie beispielsweise 80° C bis 120° C, 85° C bis 110° C oder 90° C bis 100° C, erfolgt die Freisetzung des ersten Moleküls aus dem ersten Partikel. Die Temperaturerhöhung kann ebenfalls die Anbindung des ersten Moleküls an das zweite Molekül begünstigen, wie beispielsweise im Fall der Synthese eines Peptidarrays. Das Freisetzen der ersten Moleküle aus dem ersten Partikel und gegebenenfalls das Binden des ersten Moleküls an das zweite Molekül erfolgt beispielsweise in einem Zeitraum von 30 Minuten bis 2 Stunden, vorzugsweise 1 bis 2 Stunden, wie beispielsweise 90 Minuten. Die Reaktion erfolgt gegebenenfalls unter Ausschluss von Luftsauerstoff, beispielsweise unter Argonatmosphäre oder Atmosphäre eines anderen Inertgases, wie beispielsweise eines anderen Edelgases oder Stickstoff, so dass die Reaktion von Luftsauerstoff mit dem ersten Molekül und/oder zweiten Molekül bzw. dem Oligomer im Wesentlichen vermieden wird. Vorzugsweise wird dabei eine unerwünschte Oxidation der Reaktanten bzw. Reaktionspartner weitgehend ausgeschlossen. Die Herstellung der Partikel bzw. das Binden der darin enthaltenen Moleküle und die zugrundeliegenden Bedingungen, Reagenzien, verwendeten Geräte, etc. können der WO 00/35940 entnommen werden.

Vorzugsweise erfolgt die Freisetzung des ersten Moleküls von dem ersten Partikel bzw. aus dem ersten Partikel und/oder das Binden des ersten Moleküls in der Anwesenheit eines Lösungsmittels in der Dampfphase. Das Substrat wird hierzu einer ungesättigten und/oder gesättigten Dampfatmosphäre aus dem Lösungsmittel ausgesetzt. Der Dampf kondensiert an der Oberfläche des Substrats und/oder wird von dem ersten Partikel absorbiert. Dadurch wird die Polymermatrix durchlässig, d.h. perforiert und/oder aufgelöst, und das darin enthaltene erste Molekül wird freigesetzt. Im Gegensatz zum alleinigen Freisetzen durch Temperaturerhöhung lassen sich dadurch einerseits deutlich größere Mengen an Molekülen freisetzen und andererseits können diese freigesetzten Moleküle leichter an die reaktiven Gruppen auf dem Substrat diffundieren. Bei dem Lösungsmittel handelt es sich vorzugsweise um ein oder mehrere organische Lösungsmittel. Um die Dampfatmosphäre zu generieren können beispielsweise Dichlormethan, Aceton, N,N-Dimethylformamid und/oder deren Kombinationen als organisches Lösungsmittel verwendet werden. Die Extraktion der Moleküle kann bei Temperaturen zwischen -20°C und +110°C, vorzugsweise 10 bis 80°C, mehr bevorzugt 60 bis 80°C, erfolgen. Höhere Temperaturen von bspw. >80°C bis 110°C erleichtern im Allgemeinen die Extraktion und Bindung der Moleküle. Niedrigere Temperaturen von bspw. -20°C bis <10°C verhindern die Diffusion der Moleküle während der Extraktion. Die Extraktion dauert im Allgemeinen zwischen 1 Minute und 90 Minute. Die Dauer hängt hierbei für gewöhnlich von dem verwendeten Lösungsmittel und der Temperatur ab und kann vom Fachmann ohne weiteres ermittelt werden.

Bei dem zweiten Molekül kann es sich um eine Kupplungsfunktionalität in einer Vertiefung des Substrats, wie beispielsweise eine unmittelbar auf die Substratoberfläche aufgebrachte Aminogruppe, handeln. Das erste Molekül kann anschließend an die Oberflächenfunktion binden. Alternativ kann es sich um einen in der Vertiefung des Substrats immobilisierten Abstandshalter bzw. Spacer mit daran befindlicher Kupplungsfunktionalität handeln. Weiterhin kann es sich bei dem zweiten Molekül um ein einzelnes Molekül oder mehrere Moleküle des Oligomerarrays handeln. Der Schritt des Bindens des ersten Moleküls an das zweite Molekül beschreibt somit entweder das Anbringen eines ersten Oligomermoleküls an das Substrat, unmittelbar daran oder mittelbar über einen Spacer, oder einen beliebigen Verlängerungs- bzw. Elongationsschritt des Oligomers, wie beispielsweise von einem 2mer zu einem 3mer, 10mer zu einem 11mer, oder auch 12mer zu einem 15mer, falls es sich bei dem ersten Molekül gleich um mehrere (drei) Oligomermoleküle handelt.

Vor dem Aufbau des erfindungsgemäßen Oligomerarrays kann die Oberflächenfunktionalität derart modifiziert werden, dass die physiochemischen Eigenschaften der Stege zwischen den Vertiefungen und der Vertiefungen selber variieren. Die Oberfläche der Stege zwischen den Kavitäten kann derart modifiziert werden, dass sie die Diffusion und damit das Binden des ersten Moleküls aus einer Kavität heraus zu den benachbarten Kavitäten verhindert.

Beispielsweise kann diese Modifikation der Oberfläche zwischen den Stegen durch Ätzen der Oberfläche der Stege geschehen, wobei die Vertiefungen selbst nicht dem Ätzmedium ausgesetzt werden. Die Vertiefungen können einerseits beispielsweise durch eine Polymermatrix, welche vor dem Ätzprozess in die Kavitäten eingebracht und zwecks einer homogenen Füllung gesintert wird, vor dem Ätzmedium geschützt werden. Alternativ kann die Oberfläche in den Vertiefungen durch eine aufgesputterte Metallschicht vor dem Ätzmedium geschützt werden. Hierzu wird das gesamte Substrat mit einer Metallschicht belegt, welche anschließend von der Oberfläche der Stege entfernt wird. Das Ätzen kann beispielsweise durch ein Reinigungsplasma (Sauerstoff-, Stickstoff- oder Argonplasma oder andere Gasplasmen) oder mithilfe eines flüssigen Mediums einer starken Säure oder Base erfolgen. Einzige Voraussetzung ist, dass das die Kavitäten schützende Material chemisch inert gegen das verwendete Ätzmedium ist.

Nachdem das Ätzen der Oberfläche der Stege beendet ist, wird das die Vertiefungen schützende Material aus den Vertiefungen entfernt. Die beispielsweise verwendete Polymermatrix kann hierbei durch organische Lösungsmittel entfernt werden. Eine Metallschicht kann durch anorganische Lösungsmittel entfernt werden.

Nachdem das Ätzen der Oberfläche der Stege zwischen den Kavitäten erfolgt ist, kann die Oberfläche der Stege chemisch modifiziert werden. Beispielsweise können hydrophobe Silane an die beim Ätzprozess entstandenen OH-Gruppen gekuppelt werden. Dadurch kann die Diffusion der Moleküle zwischen den einzelnen Kavitäten während der Extraktion verhindert werden.

Neben der Möglichkeit des Ätzens besteht die Möglichkeit, die Oberfläche der Stege durch die Aktivierung von fotosensitiven Gruppen zu modifizieren. Hierzu wird auf die Oberflächenfunktionalität des gesamten Substrats ein fotosensitiver Linker gekuppelt, welcher durch Belichten mit UV-Licht abgespalten werden kann. Diese Belichtung kann beispielsweise mithilfe eines sog. Mask-Aligners, wie er in der Halbleiterfertigung bekannt ist, erfolgen. Nach dem Abspalten des Linkers auf den Stegen zwischen den Kavitäten kann diese Oberfläche hydrophobiert werden, wodurch die Diffusion der Moleküle zwischen den einzelnen Vertiefungen während der später stattfindenden Extraktion verhindert wird. Im Anschluss an die Hydrophobierung wird der fotosensitive Linker in den Kavitäten abgespalten. Im Anschluss kann Schritt (b) Einbringen eines ersten Partikels mit einem ersten Molekül in eine Vertiefung erfolgen und die weiteren Schritte durchgeführt werden.

Während des Schritts des Freisetzens des ersten Moleküls von dem ersten Partikel und/oder während des Schritts des Bindens des ersten Moleküls an ein zweites Molekül unter Bildung eines Oligomers können die Vertiefungen abgedichtet werden. Dies verhindert die Diffusion des ersten Moleküls bzw. der ersten Moleküle aus den Vertiefungen und eine damit einhergehende Kontamination anderer Vertiefungen bzw. eine geringere Umsatzrate. Das Abdichten kann beispielsweise durch das Aufbringen eines Dichtmaterials auf die Substratoberfläche erreicht werden. Die Dichtung kann durch den Kontakt des Dichtmaterials und der Substratoberfläche sichergestellt werden. Folglich stellt jede Kavität ein abgeschlossenes System dar, in der die Freisetzung und/oder das Binden des Moleküls bzw. der Moleküle vollkommen unabhängig von den anderen Kavitäten des Substrats stattfinden. Als Dichtmaterial kommen beispielsweise PDMS, PTFE, PFDV-Membranen oder andere kommerziell erhältliche Klebebänder in Frage. Alternativ oder zusätzlich kann jeder andere Verfahrensschritt, bei dem eine Isolierung von der Umgebung gewünscht ist, entsprechend, d.h. unter Abdichtung der Vertiefung, durchgeführt werden.

Nach dem Ablagerungsschritt wird für jede Ebene der Synthese die Lage der verschiedenen Monomerpartikel bestimmt. Dies geschieht vorzugsweise dadurch, dass die Partikel zuvor mit Fluoreszenzfarbstoffen bzw. Lumineszenzfarbstoffen codiert werden, so dass ein CMOS-Sensor, eine CCD-Kamera oder ein Fluoreszenzscanner dazu verwendet werden kann, um herauszufinden, in welcher Kavität sich welches Partikel befindet. Die Codierung der verschiedenen ersten Moleküle und/oder ersten Partikel kann durch die unterschiedlichen Fluoreszenzfarbstoffe bzw. Lumineszenzfarbstoffe, durch unterschiedliche Konzentrationen derselben und durch eine Kombination gegebenenfalls verschieden konzentrierter Farbstoffe erfolgen. So können beispielsweise drei verschiedene Farbstoffe in zwei unterschiedlichen Konzentrationen sechs verschiedene Partikel codieren, bzw. wenn diese auch noch miteinander kombiniert werden, so können 6 x 6 = 36 verschiedene Partikel codiert werden.

Die Verwendung der ersten Partikel mit (jeweils) einem ersten Molekül ermöglicht die Herstellung von Arrays mit ultra-hohen Spotdichten, wie beispielsweise 106 Spots/cm² oder mehr. Die ersten Partikel können über einfache Wischtechniken in entsprechende Vertiefungen verbracht werden, so dass eine gezielte Ablegung der ersten Partikel bei dem erfindungsgemäßen Verfahren nicht notwendig ist. Hierdurch unterscheidet sich das erfindungsgemäße Verfahren von den Verfahren gemäß dem Stand der Technik, bei denen eine gezielte Ablegung der Partikel erforderlich ist.

Das Syntheseverfahren kann analog zur Festphasensynthese den vereinfachten Einbau von Oligomeren bzw. modifizierten Oligomeren und Reaktionen derselben ohne Verlängerung des Oligomers umfassen. Beispielsweise können in Aminosäuren Funktionalitäten, das heißt reaktive Gruppen, gezielt zur Reaktion gebracht werden, so dass an dieser Stelle, die nicht dem Rückgrat (Backbone) des Oligomers entspricht, beispielsweise eines oder mehrere weitere Moleküle eingebaut werden können oder eine Zyklisierung innerhalb des Oligomers erfolgt. Es ist klar, dass eine entsprechende Schutzgruppenchemie angewendet werden muss, um die gezielte Reaktion der Moleküle zu ermöglichen und unerwünschte Reaktionen auszuschließen. Hierfür ist im Stand der Technik eine Vielzahl von Schutzgruppen bekannt, die gezielt aufgebracht und wiederum entfernt werden können.

Der detektierbare Marker kann durch ein geeignetes Verfahren visualisiert werden, um beispielsweise eine 3D-Ablagerungsmaske des Oligomerarrays zu erstellen. Weiterhin können bildgebende Verfahren zum Einsatz gelangen, die jegliche Art von Vorrichtung oder Gerät umfassen, mit dem ein Bildsignal oder eine Abbildung in Reaktion auf ein Erfassen eines detektierbaren Markers erzeugt werden kann. Die Vorrichtung ermöglicht die flächige Lokalisierung des detektierbaren Markers auf dem Substrat und somit eindeutige Bestimmung des Moleküls in Bezug auf dessen Position innerhalb des Oligomers und Vertiefung in dem Substrat.

"Abstandshalter" bzw. "Spacer" oder "Linker", wie sie beispielsweise bei den erfindungsgemäßen Oligomerarray bzw. Verfahren zu deren Herstellung verwendet werden, sind dadurch gekennzeichnet, dass sie ein erstes Ende aufweisen, das in der Vertiefung des Substrats an die Substratoberfläche gebunden ist, und ein zweites Ende, das an das Oligomer bzw. an ein Oligomermolekül gebunden ist. Der Abstandshalter trennt daher das Substrat von dem Oligomer bzw. Oligomermolekül, ist jedoch mit beiden verbunden. Der Abstandshalter ist hierbei endständig kovalent mit dem Substrat und dem Oligomer bzw. Oligomermolekül verbunden.

Abstandshalter können unmittelbar auf der Substratoberfläche synthetisiert werden, gefolgt von der Erzeugung einer kovalenten Bindung an erste Molekül, oder als Ganzes mit Substrat und anschließend mit dem ersten Molekül verbunden werden. Bindungen in dem Abstandshalter können C-C-Einfachbindungen, C-C-Doppelbindungen, C-N-Einfachbindung, oder C-O-Einfachbindungen umfassen. Der Abstandshalter kann darüber hinaus Seitenketten oder andere Substituenten umfassen. Substrat und erstes Molekül können mittels einer geeigneten Reaktion mit dem Abstandshalter verbunden werden, um so eine kovalente Bindung dazwischen zu erzeugen. Geeignete Abstandshalter umfassen Alkyl, Alkynyl, Alkynyl-Ketten, aromatische, polyaromatische, und heteroaromatische Ringe, wobei jedes davon weiter substituiert vorliegen kann. Spacer weisen vorzugsweise einen linearen C-C Grundkörper einer Länge von 10 bis 25 Kohlenstoffatomen, vorzugsweise 12 bis 18 Kohlenstoffatomen, wie etwa 15 Kohlenstoffatomen auf. Alternativ können Oligomerbausteine als Spacer benutzt werden. Beispielsweise kann ein 15mer Polyglycin als Spacer für einen Peptidarray zur Anwendung gelangen. Es versteht sich, dass die Länge der Spacer und deren Eigenschaften nach Bedarf frei gewählt werden können. Geeignete Spacer, deren Herstellung und deren Reaktion mit Substrat und/oder einem Molekül eines aufzubauenden Oligomers sind dem Fachmann bekannt.

Die Abstandshalter können derart ausgewählt werden, dass ein guter Zugang des Bindepartners zu dem Oligomer ermöglicht wird und/oder das Oligomer frei an der Substratoberfläche beweglich ist. Weiterhin kann der Spacer derart ausgestaltet sein, dass eine chemische Spaltung an oder in dem Spacer ermöglicht wird, um dadurch die, ggf. gezielte Abspaltung, eines bestimmten Oligomers zu ermöglichen. Dies hat den Vorteil, dass das Oligomer ohne Behinderung durch das Substrat untersucht werden kann.

Es besteht die Möglichkeit, abspaltbare Abstandshalter in die Synthese des Oligomerarrays mit einzubeziehen. Dadurch können synthetisierte Oligomere einfach abgespalten und auf eine Zieloberfläche transferiert werden. Dies ermöglicht ferner eine Aufreinigung der Oligomere, da lediglich vollständig synthetisierte Oligomere transferiert werden. Die Abbruchprodukte (nicht vollständig synthetisierte Oligomere) hingegen werden nicht transferiert und können während eines Waschschritts entfernt werden. Vollständige Oligomere sind diejenigen Oligomere, welche eine Kettenlänge entsprechend der Anzahl der durchlaufenden Durchgänge gemäß dem vorliegenden Verfahren aufweisen, d.h. der Zahl der Monomere des zu erzielenden Oligomers. Zudem können die Oligomerarrays durch den Transfer vervielfältigt werden. Durch Steuern der prozentualen Abspaltrate können somit mehrere Replikate desselben Oligomerarrays erreicht werden.

Die Abspaltung der Moleküle wird durch dem Einbau abspaltbarer Abstandshalter an der Basis der Oligomere erreicht. Solche durch beispielsweise Licht, insbesondere UV-Licht oder Hydrogenolyse oder Photolyse oder unter basischen Bedingungen usw. sowie gegenüber den Reaktionsbedingungen einer Synthese stabiler abspaltbarer Abstandshalter sind dem Fachmann bekannt. Selektiv spaltbare Linker, die auf einer Methionin- und einer Estergruppe basieren können bspw. der DE 69435011 T2 entnommen werden. Fields GB und RL Noble, 1990, Solid phase peptide synthesis utilizing 9-fluorenylmethoxycarbonyl amino acids, Int. J. Pept. Protein Res. 35: 161-214 offenbart weitere derartige Linker. Beispiele weiterer Abstandshalter sind der Rink Amide Linker oder UV spaltbarer Linker, die M.S. Bernatowicz, S.B. Daniels, H. Köster, Tetrahedron Lett. 30 (1989) 4645 bzw. Stefan Peukert and Bernd Giese, The Pivaloylglycol Anchor Group: A New Platform for a Photolabile Linker in Solid-Phase Synthesis, J. Org. Chem. 1998, 63, 9045-9051 entnommen werden können.

Für den Transfer werden die Kavitäten mit einer Pufferlösung befüllt. Die Zusammensetzung des Puffers hängt von der Beschaffenheit der synthetisierten Oligomeren ab und ist dem Fachmann geläufig. Um die beim Transfer auftretende Diffusion der Oligomere aus einer Kavität heraus in benachbarte Kavitäten zu verhindern, kann als Zieloberfläche beispielsweise eine Membran genutzt werden, welche die einzelnen Kavitäten abdichtet. Nach dem Platzieren der Membran erfolgt die Abspaltung des Abstandshalters. Sämtliche Oligomere (komplett synthetisierte Oligomere sowie Abbauprodukte) können sich nun in den Grenzen der Diffusion frei in der mit der Pufferlösung befüllten Kavitäten bewegen.

Um nur die komplett synthetisierten Oligomere zu transferieren und damit eine Aufreinigung des Oligomerarrays zu erzielen, wurde in einem letzten Schritt der Oligomersynthese eine funktionelle Gruppe an die endständigen Gruppen der Oligomere gekuppelt. Diese kann bspw. entsprechend der Festphasensynthese nach Merrifield nur an komplett synthetisierte Oligomere kuppeln, da die Abbauprodukte bereits acetyliert (blockiert) worden sind. Diese funktionellen Gruppen können mit der Zieloberfläche, welche ebenfalls funktionalisiert ist, eine Bindung eingehen. Beispiele dieser Bindungen sind Biotin-Streptavidin, Azid-Alkin oder Thiol-Gold Wechselwirkungen. Nach der Abspaltung der Syntheseprodukte werden nur komplett synthetisierte Oligomere die Bindung mit der funktionalisierten Zieloberfläche eingehen, während die Abbauprodukte weggewaschen werden.

Im Vergleich zu den seither bekannten Verfahren gemäß dem Stand der Technik können sogenannte "fokussierte" Oligomere bzw. Oligomerbibliotheken einfacher synthetisiert werden. Beispielsweise können an beliebigen Kupplungsebenen, wie beispielsweise die Kupplungsebene 5, 8, 9, 10, 11, von an dem in der Vertiefung des Substrats angebrachten bzw. immobilisierten C-Terminus des Oligomers an gerechnet, mit jeweils sortenreinen Partikeln, das heißt ausschließlich das erste Molekül beinhaltende Partikel, oder mit eingeschränkten Gemischen, wie beispielsweise zwei Partikeln mit jeweils einem verschiedenen ersten Molekül, umgesetzt werden, wohingegen bei den anderen Kupplungsebenen eine Vielzahl von Varianten generiert werden. Mit derartig fokussierten Oligomerbibliotheken können infolge eine Vielzahl von Oligomervarianten hergestellt werden, die alle ein bestimmtes Epitop bzw. Strukturmerkmal enthalten, die dann gezielt auf die Bestimmung eines geeigneten Bindepartners untersucht werden können. Da das Epitop hierbei bereits eine Bindung an den Bindepartner ermöglicht, kann durch die Variation der übrigen Moleküle innerhalb der entsprechenden Monomere gegebenenfalls eine verbesserte Bindung an den Bindepartner hervorgebracht werden.

Als Beispiel kann das 15 Aminosäuren lange Flag-Epitop (XXXXDYKD/EXXDXXXX) herangezogen werden. Entsprechende Peptidvarianten können hergestellt werden, die alle an den Flag-M1-Antikörper binden und diese nach solchen Peptiden untersuchen, deren Bindung an den Antikörper zum Beispiel durch veränderte Temperaturen, oder Licht gezielt beeinflusst, beispielsweise schaltbar, ist.

Somit werden neuartige Anwendungen für Oligomere, wie beispielsweise Peptide, im Arrayformat zugänglich, die beispielsweise in der Forschung und/oder Diagnostik (Auslesen von Antikörperbindungen oder Antikörperprofilen von Patienten) oder in der Pharmaindustrie (zirkuläre Peptide eignen sich insbesondere für die Suche nach Bindern für Proteine und für Therapeutika, sowie für das Auslesen der Bindeepitope von Antikörper, die konformationelle Epitope erkennen).

Die Substrate bzw. Träger der Arrays können aus verschiedenen Materialien wie Glas, Quarz, Silizium, Keramik, Quarz, Silizium, Keramik, Kunststoff, Metalle und/oder Kompositwerkstoffen hergestellt werden. Alle Materialien sind hierbei gegen die Chemikalien beständig, die bei der kombinatorischen Synthese verwendet werden. Dies sind bei der Peptidsynthese vorzugsweise die Lösungsmittel N-Methyl-2-Pyrrolidon (NMP), Dimethylformamid (DMF), Aceton, Dichlormethan, Ethanol, Wasser sowie Diispropylamin und Essigsäureanhydrid. Für das Abspalten der Peptide mindestens 50 % Trifluoressigsäure (TFA) in einem geeigneten Lösungsmittel, wie bspw. Isopropanol, Methanol und Dichlormethan. Außerdem müssen die Materialien die Mikrostrukturierung, das heißt das Erstellen geeigneter Vertiefungen, erlauben. Die Mikrostrukturierung der Glasoberflächen kann beispielsweise mit gängigen lithografischen Verfahren, zum Beispiel mittels eines Trocken- oder Nassätzprozesses, erfolgen. Vorteile der Kunststoffmaterialen bestehen in der Möglichkeit zur schnellen Vervielfältigung der mikrostrukturierten Substrate zum Beispiel mittels Heißprägen. Geeignete Materialien sind dem Fachmann geläufig.

Die Vielfalt der möglichen Materialien ergibt sich aus der Tatsache, dass das Befüllen der Vertiefungen mit Monomer-enthaltenden Partikeln keine Transparenz und keinerlei Positionierung der Substrate verlangt. Vorzugsweise handelt es sich bei dem Substrat um ein im Wesentlichen nicht-transparentes bzw. undurchsichtiges Material. Lediglich die Möglichkeit der Detektion der abgelagerten Partikel nach der Deposition, das heißt dem Einbringen in eine bestimmte Vertiefung, sowie die Derivatisierung der Oberfläche mit funktionellen Gruppen für eine Festphasensynthese ist von Bedeutung. Monomer-enthaltende Partikel bzw. erste Partikel mit einem ersten Molekül, können durch unterschiedliche Verfahren hergestellt werden. Die Funktion der Partikel besteht darin Monomerbausteine, bzw. erste Moleküle, für die kombinatorische Synthese zum Syntheseort zu transportieren.

Beispielsweise können Polymerpartikel mit eingebetteten Monomeren, die sich beim Schmelzen der Polymermatrix an der Kupplungsreaktion an der Oberfläche beteiligen können, verwendet werden. Derartige Partikel können mittels Emulsions-, Mal- oder Sprühtrocknungsverfahren hergestellt werden. Beide Verfahren ermöglichen Partikelabmessungen im Mikrometerbereich. Um eine möglichst schmale Größenverteilung der Partikel zu erhalten, können nach der Partikelherstellung weitere Prozessschritte wie Sieben oder Sichten angeschlossen werden. Entsprechende Siebe und Sichter sind im Stand der Technik bekannt. Sollen Partikel im Submikrometerbereich hergestellt werden, geraten Sieb- und Sichtungsverfahren im Allgemeinen an ihre Grenzen. Dies kann umgangen werden, indem der während des Sprühtrocknungsprozesses unausweichlich vorhandene Anteil von feinen Partikeln genutzt werden, die einen Durchmesser von weniger als 1 µm aufweisen. Aufgrund der angepassten Größe der Vertiefungen finden anschließend nur diese feinen Partikel den Weg in die Vertiefungen. Alle größeren Partikel werden automatisch beim Auftragen bzw. Aufbringen der Partikel auf die Substratoberfläche, wie beispielsweise durch Einbringen der Molekül-enthaltenen Partikel auf die Substratoberfläche, von der Substratoberfläche entfernt. Gewöhnliche Sichtungsprozesse sind für gewöhnlich bei der Herstellung dieser feinen Partikel nicht ohne weiteres zu verwenden.

Alternativ können auch Kompositpartikel verwendet werden, die einen bereits mit anderen Verfahren hergestellten Kern aufweisen und deren Oberfläche mit Monomeren bzw. Molekülen belegt ist. Beispielsweise kann ein anorganischer Kern aus Siliziumdioxid bereitgestellt werden, der mit einer Schale aus der Polymermatrix mit den eingebetteten Monomeren bzw. Molekülen umhüllt ist. Ein Vorteil dieser Partikel besteht in ihrer Monodispersität und ihrer sphärischen Form.

Die Herstellung von Partikeln mit regelmäßigen Abmessungen kann auch mit Hilfe von mikrostrukturierten Oberflächen erreicht werden. Die Vertiefungen werden mit Festsubstanzen, wie beispielsweise polydispersen Polymerpartikeln, befüllt und anschließend erhitzt, so dass die Substanz die Form der Vertiefung annimmt. Die Entfernung der Partikel kann beispielsweise mit Hilfe von Ultraschall erfolgen. Alternativ können die Vertiefungen mit flüssigen Materialien befüllt werden, wobei die Moleküle/Monomere sowie die Partikelmatrix in Lösungsmitteln, wie beispielsweise Dichlormethan, gelöst werden. Nach dem Trocknungsschritt haben sich in den Vertiefungen gleichförmige Monomer-haltige Partikel ausgebildet.

Die vorliegende Erfindung betrifft im Wesentlichen drei Varianten zur zufälligen Ablagerung von Partikeln. Einerseits können Partikelgemische zufällig in die Vertiefungen eingebracht werden, wobei die unterschiedlichen Partikel markiert sind. Alternativ dazu kann eine sequenzielle zufällige Partikelablagerung ohne Markierung der Partikel erfolgen. Weiterhin ist eine Kombination der beiden vorstehend erwähnten Varianten möglich.

Bei der ersten Variante wird die Markierung der Partikel mit Farbstoffen, Fluoreszenz-Markern, oder über die Form der Partikel vorgenommen. Dies kann beispielsweise aber auch dadurch erreicht werden, dass die Monomerpartikel lumineszieren oder mit beispielsweise Oligonukleotid-Markern versehen sind, die mittels Hybridisierung nachgewiesen werden können. Derartige Techniken sind dem Fachmann gut bekannt. Wichtig für die Positionsbestimmung ist dabei nur, dass sich jede Sorte der Monomerpartikel von anderen Monomerpartikeln eindeutig unterscheiden lässt. Wenn diese Bedingung erfüllt ist, werden alle Sorten der Partikel vermischt und im Überschuss auf die Oberfläche mit den Vertiefungen aufgebracht. Alle Vertiefungen werden anschließend zufällig mit Partikeln befüllt, indem die Partikel in trockenem Zustand eingerieben werden oder mittels einer Trägerflüssigkeit in die Vertiefungen verbracht werden. Durch Auslesen der Markierungen wird anschließend festgestellt, welches Monomer bzw. welches Partikel in welcher Vertiefung abgelagert ist. Die Variante 1 hat hierbei den Vorteil, dass sämtliche für eine Lage notwendige Monomerpartikel in einem einzigen Schritt, und damit sehr schnell, sehr verlässlich (weil ein Überschuss an Partikeln eingesetzt werden kann) und sehr einfach auf die Substratoberfläche aufgebracht werden.

Bei der Variante 2 werden die Partikel zufällig auf die Substratoberfläche mit den Vertiefungen aufgebracht, wobei lediglich ein Teil der Vertiefungen befüllt wird. Das sequenzielle Befüllen der Vertiefungen mit unterschiedlichen Monomerpartikeln führt zum kompletten Befüllen im Wesentlichen aller Vertiefungen. Durch die Detektion der Ablagerungsorte jenes Monomerpartikels wird nach dem Befüllen der Vertiefungen eindeutig die Information ermittelt, welches Monomer in welcher Vertiefung abgelagert wurde. Bei zwanzig Monomeren müssen somit zwanzig Detektionsdurchläufe vorgenommen werden. Die Variante 2 weist hierbei den Vorteil auf, dass sie keine Markierungen der Partikel und/oder der ersten Moleküle benötigt. Allerdings kann eine Markierung der Partikel mit bspw. einem Fluoreszenmarker die Bestimmung der Ablagerungsposition erleichtern.

Weiterhin eignet sich zur zufälligen Ablagerung auch eine Kombination beider Varianten. Wenn dabei drei Marker für die Partikelmarkierung genutzt werden, reduziert sich die Zahl der zufälligen Ablagerungsschritte um den Faktor 3. Neben der Oberfläche mit beispielsweise zylinderförmigen Vertiefungen können auch andere Geometrien, wie beispielsweise Waben oder Kanäle, für die Partikelablagerung genutzt werden.

Markierte Partikel können durch eine Vielzahl verschiedener Verfahren hergestellt werden. Im Allgemeinen können Partikel durch Verwendung einer Luftstrahlmühle, Sprühtrocknung, Gießen bzw. Schmelzen in Formen hergestellt werden. Die Markierung kann bereits während der Herstellung oder auch im Anschluss daran erfolgen. Farbstoffe, fluoreszierende oder lumineszierende Moleküle oder Nanopartikel, wie beispielsweise Quantumdots, können bereits während der Herstellung der Partikel zugegeben werden, die hierfür vorzugsweise zwei Eigenschaften aufweisen sollten: Sie sollten die Festphasensynthese nicht stören (beispielsweise sollten die beigefügten Markierungs*agenzien bei* der Peptidsynthese keine freien Aminogruppen oder SH-Gruppen aufweisen, die mit den C-terminalaktivierten Monomer-Bausteinen zur Reaktion kommen) und außerdem sollten sie die Bildung der Partikel nicht stören (beispielsweise können metallische oder keramische Nanopartikel die Düsen eines Sprühtrockners verstopfen). Geeignete Farbstoffe sind dem Fachmann bekannt.

Diese Bedingungen gelten jedoch nur bis zu einem gewissen Grad bzw. überhaupt nicht, da für die Markierung der Partikel unter Umständen nur sehr geringe Mengen, beispielsweise an Fluoreszenzfarbstoffen, benötigt werden, so dass es vorteilhaft sein kann, gezielt an in großem Überschuss vorliegende C-terminalaktivierte Aminosäurebausteine zu kuppeln, um die Fluoreszenzfarbstoffe in einem nachgelagerten Schritt an bereits vorgeformte Partikel zu binden. Dies kann als Beispiel dafür dienen, das vorgeformte Monomerpartikel auch in einem nachgeschalteten Prozess markiert werden können, indem die Partikel beispielsweise kurz in einer gegebenenfalls erwärmten wässrigen und/oder alkoholischen Lösung, beispielsweise Ethanol, inkubiert werden, die auch den entsprechenden Farbstoff enthält.

Die Ablagerung der Partikel kann auf viele verschiedene Arten erfolgen, die dem Fachmann bekannt sind. Ein besonders einfaches und bevorzugtes Verfahren verwendet dabei trockene Partikel, die mit einem fusselfreien Papier in die Vertiefungen des Substrats eingerieben werden. Wenn die Partikel hierbei im Überschuss vorhanden sind, so lässt sich dadurch eine nahezu 100 %ige Belegungsrate erreichen, das heißt, in mindestens 90 %, vorzugsweise 95 % oder mehr, 98 % oder mehr oder 99,5 % oder mehr, der Vertiefungen befindet sich jeweils ein Partikel. Wenn die Partikel eine mit der Vertiefung abgestimmte Größe haben, das heißt, dass lediglich ein Partikel in einer Vertiefung platziert werden kann, kann dadurch auch ohne Weiteres erreicht werden, dass in jeder Vertiefung danach ein Partikel abgelagert wird. Ein Abstimmen der Größe ist leicht zu erreichen: Die Partikel müssen so klein sein, dass ein Partikel in eine Vertiefung passt und sie müssen so groß sein, dass zwei Partikel nicht mehr in eine Vertiefung passen. Alternativ können die Partikel auch in einer Flüssigkeit suspendiert und in die Vertiefungen eingerieben werden.

Die nahezu vollständige und sehr einfache Ablagerung der Partikel aus Flüssigkeiten in Mikro- und sogar Nanokavitäten funktioniert bis in Dimensionen von einigen hundert Nanometern Partikeldurchmesser, insbesondere wenn ein sich über die Oberfläche der Kavitäten zurück ziehender genau definierter Meniskus aus Luft die Partikel regelrecht in die Kavitäten treibt. Das Einbringen bzw. die Ablagerung von Partikeln in Kavitäten bzw. Vertiefungen verschiedener Größe ist beispielsweise in Yadong Yin et al., Template-Assisted Self-Assembly: A Practical Route to Complex Aggregates of Monodispersed Colloids with Well-Defined Sizes, Shapes, and Structures, J. Am. Chem. Soc. 2001, 123, 8718-8729 gezeigt.

Die Ablagerung der Partikel mit unterschiedlicher Belegungsrate lässt sich entweder durch einstellen der Partikelkonzentration in einer Lösung oder einem Aerosol oder auch durch die Verwendung spezieller Walzen realisieren. Durch Konzentrationsänderungen der Partikelmasse in einer Lösung lassen sich Belegungsraten von 0,01 - 100 %, wie beispielsweise 1 - 99 %, 5 - 95 %, 10 - 90 %, 20 - 80 %, 30 bis 70%, 40 bis 60 %, bzw. 45 - 55 % erreichen.

Durch eine Mikrostrukturierung der Walze wird die Kontaktfläche zwischen der Walze und dem Substrat verringert. Nur die Partikel an der Kontaktfläche werden übertragen, wohingegen Partikel, die keinen Kontakt zum Substrat haben, auf der Walze verbleiben. Die Belegungsrate lässt sich somit durch die Anpassung des Walzenreliefs beliebig anpassen. Die Elastizität der Walze kann dabei die Qualität der Ablagerung beeinflussen: Bei einer elastischen Walze werden Partikel, die auf einer glatten Oberfläche liegen, immer auf der Walzenoberfläche haften bleiben, weil durch den Druck der Walze die Kontaktfläche zwischen selbiger und dem Partikel zunimmt. Auf der anderen Seite verlieren diejenigen Partikel, die in den Vertiefungen liegen, den Kontakt zur Walze und verbleiben folglich in den Vertiefungen.

Zur Detektion der Partikelablagerung werden Methoden verwendet, welche die Markierung der abgelagerten Partikel möglichst einfach und möglichst schnell erkennen können. Dabei können hochauflösende Kamerasysteme, Scanner, Lumineszenz- und Fluoreszenzscanner oder Scanning-Mikroskope benutzt werden. Der große Vorteil des Kamerasystems ist die schnelle Detektion der abgelagerten Partikelmuster. In diesen Fall könnte ein Kamerasystem mit einem großen Gesichtsfeld eingesetzt werden, welches zugleich die benötigte Auflösung zur sicheren Detektion der einzelnen Spots aufweist. Z.B. erlauben auf dem Markt vorhandene Kamerachips mit bis zu 200 Megapixeln mehr als 100 Pixel pro Spot, wenn 1 cm² bei einem Abstand zweier Vertiefungsmittelpunkte bzw. Pitch von 10 µm abgebildet wird. Bestimmt werden können dabei die Form, die Farbe, die zeitliche Abfolge der Ablagerung, die Fluoreszenz oder die Lumineszenz der Partikel, die Stärke der jeweiligen Signale und alle denkbaren Kombinationen dieser Eigenschaften. Wichtig ist nur, dass diese Methode die jeweiligen Orte und die unterschiedlichen Identitäten der Partikel verlässlich erkennt

Bei der Markierung der Schutzgruppen mit Farbstoffen haben die Syntheseorte Abstände voneinander, die um Größenordnungen kleiner als Wellenlänge sind, und können dadurch nur mit speziellen Fluoreszenztechniken ermittelt werden. Hierfür kann beispielhaft Superresulutionsmikroskopie wie PALM, STORM und dSTORM verwendet werden. Der Abstand zwischen zwei benachbarten Spots kann durch die Konzentration der Monomere kontrolliert werden.

Es gibt bereits eine ganze Reihe bereits bekannter Anwendungen von Peptidarrays. Dazu zählen parallelisierte Diagnosen. Andere bereits bekannte Anwendungen zerlegen die Proteinsequenzen eines bekannten antigenen Proteins in einander überlappende Peptide, um herauszufinden, an welche Substrukturen des Proteins (Epitope) ein monoklonaler oder polyklonaler Antikörper bindet.

Es besteht ferner die Möglichkeit, stochastische Assays mit den erfindungsgemäßen stochastischen Oligomerarrays durchzuführen. Hierzu wird ein abspaltbarer Linker zwischen dem Oligomer und der Vertiefung eingefügt, d.h. bei dem zweiten Molekül handelt es sich um den abspaltbaren Linker. Beipsielsweise kann mithilfe der stochastischen Assays die Wirkung von Peptiden auf Baktierien oder Proteine untersucht werden.

Hierzu kann nach der Synthese des Oligomerarrays eine die zu untersuchende Probe auf den Array gegeben. Bei der Probe handelt es sich um eine flüssige Probe, wie beispielsweise eine Lösung mit Bakterien, gelösten Proteinen oder andere zu untersuchende Proben. Die Vertiefungen werden mit der Lösung befüllt. Ein Überschuss an Lösung kann entfernt werden, so dass aufgrund der Oberflächenspannung, von bspw. wässrigenLösungen, in der Vertiefung ein in Richtung der Öffnung der Vertiefun gewölbtes Flüssigkeitsreservoir (konkaver oder konvexer Meniskus) ausgebildet wird. Wird nun ein Deckplättchen, beispielsweise aus Glas, in einem definierten Abstand über dem Substrat positioniert, so gehen die Flüssigkeitsreservoire eine Verbindung mit dem Deckplättchen ein. Anschließend können die abspaltbaren Linker durch beispielsweise UV-Licht abgespalten werden und die Wirkung der abgespaltenen Oligomere auf die Baktierien, die Proteine oder den eingebrachten Stoff untersucht werden. Das Deckplättchen erfüllt hierbei zwei Funktionen. Zum Einen wird durch die plane Oberfläche eine Detektion der zu untersuchenden Wechselwirkungen ermöglicht. Zum anderen verringert das Deckplättchen durch seinen geringen Abstand zum Substrat eine Verdunstung der eingebrachten Lösung.

Der Ausdruck "aufweisen" bzw. "aufweisend" bezeichnet im Rahmen der vorliegenden Erfindung eine offene Aufzählung und schließt neben den ausdrücklich genannten Bestandteilen bzw. Schritten andere Bestandteile bzw. Schritte nicht aus. Wenn im Rahmen der vorliegenden Erfindung eine Zusammensetzung unter Verwendung des Ausdrucks "aufweisen" bzw. "aufweisend" beschrieben ist, schließt dies ausdrücklich Zusammensetzungen ein, die aus den genannten Bestandteilen bestehen oder im Wesentlichen aus den genannten Bestandteilen bestehen.

Der Ausdruck "bestehen aus" bzw. "bestehend aus" bezeichnet im Rahmen der vorliegenden Erfindung eine geschlossene Aufzählung und schließt neben den ausdrücklich genannten Bestandteilen bzw. Schritten jegliche anderen Bestandteile bzw. Schritte aus.

Der Ausdruck "im Wesentlichen bestehen aus" bzw. "im Wesentlichen bestehend aus" bezeichnet im Rahmen der vorliegenden Erfindung eine teilweise geschlossene Aufzählung und bezeichnet Zusammensetzungen, die neben den genannten Bestandteilen nur noch solche weitere Bestandteile aufweisen, die den Charakter der Zusammensetzung nicht materiell verändern oder die in Mengen vorliegen, die den Charakter der Zusammensetzung nicht materiell verändern.

Weitere Merkmale und Vorteile der Erfindung ergeben sich aus der nachfolgenden Beschreibung bevorzugter Ausführungsbeispiele unter Bezugnahme auf die Zeichnungen. Es zeigen:
- Fig. 1: eine schematische Darstellung der Verfahrensschritte zur Herstellung eines Peptidarrays;
- Fig. 2: eine schematische Darstellung eines Einbringens verschiedener erster Partikel mit jeweils einem ersten Molekül in eine Vertiefung eines Substrats und Freisetzen des ersten Moleküls von dem ersten Partikel;
- Fig. 3: Ablagerungsmethoden für reduzierte Belegungsraten: Minimierung der Konzentration der zugefügten Partikel in Lösung (links) und die stochastische Aufbringung durch eine mikrostrukturierte Walze (rechts);
- Fig. 4: das 60 %ige Befüllen zylindrischer Vertiefungen (Durchmesser 5 µm, Pitch 10 µm, Tiefe 5 µm) mit Polymerpartikeln (Durchmesser 4µm);
- Fig. 5: 5 %iges Befüllen von zylindrischen Vertiefungen (Durchmesser 6 µm, Pitch 4 µm, Tiefe 5 µm) mit Partikeln (Durchmesser 4,21 µm). Die Partikel wurden in Suspension in die Vertiefungen eingebracht, die Konzentration der Suspension beträgt 2 µl Partikel in 2,4 ml Wasser;
- Fig. 6: 6,4 %ige Befüllen von zylindrischen Vertiefungen (Durchmesser 0,6 µm, Pitch 2 µm, Tiefe 1 µm) mit Glycin-Aminosäurepartikel. Das Partikelmuster wurde mit Hilfe einer Walze hergestellt;
- Fig. 7: ein Beispiel zur Markierung von Monomerpartikeln mit Fluoreszenz-Farbstoffen: Lichtmikroskopaufnahme (Fig. 7a) und Fluoreszenzbild der zufällig abgelagerten Partikel (Fig. 7b). Die Lage der Monomere wird aufgrund des Fluoreszenzsignals identifiziert (rot, grün). Ungefüllte Vertiefungen werden für die Ablagerung nachfolgender Monomere benutzt. Pitch 10 µm;
- Fig. 8: den fluoreszenzmarkierten Dipeptid-Array nach Beispiel 2 mit zufälliger Anordnung der Monomere;
- Fig. 9: die Auswertung der Dipeptidsynthese nach Fig. 8;
- Fig. 10: den Peptidarray nach Beispiel 3. Die jeweils linke Abbildung der Fig. 10a bis 10e zeigt für jede der neun möglichen Schichten die Fluroeszenzaufnahme. Die rechte Abbildung zeigt für die jeweilige Schicht das dazugehörige Aminosäuremuster;
- Fig. 11: das Fluoreszenzmuster des mit Anti-HA- und Anti-FLAG-Antikörpern inkubierten stochastischen Peptidarrays aus Beispiel 3; und
- Fig. 12: das verarbeitete Bild des Fluoreszenzmusters des mit Anti-HA- und Anti-FLAG-Antikörpern inkubierten stochastischen Arrays.

In einer Ausführungsform ist das erste Molekül eine Aminosäure und/oder ein Aminosäurederivat.

Die Aminosäure/ das Aminosäurederivat reagiert mit dem zweiten Molekül unter Ausbildung eines Amids. Das zweite Molekül kann ebenfalls eine Aminosäure und/oder ein Aminosäurederivat sein, ebenso kann das zweite Molekül eine Oberflächenfunktionalität auf der Substratoberfläche, insbesondere in der Vertiefung des Substrats, sein bzw. eine Funktionalität an einem Ende eines Spacers, der an seinem anderen Ende in einer Vertiefung eines Substrats immobilisiert vorliegt. Für gewöhnlich stellt die Reaktion des ersten Moleküls mit dem zweiten Molekül eine Kupplungsreaktion unter Ausbildung einer Amidbindung dar. Gleichermaßen handelt es sich bei der Oberflächenfunktionalität oder bei der Funktionalität an einem Ende eines Spacer für gewöhnlich um eine Carboxygruppe, die mit dem N-Terminus des ersten Moleküls zu einem Amid reagiert. In Folge wird auf der Substratoberfläche entweder unmittelbar oder mittelbar über einen Spacer ein Peptid aufgebaut. Das Peptid wird vorzugsweise gemäß der Peptidfestphasenchemie erzeugt, die dem Fachmann geläufig ist.

Aminosäuren bzw. Aminosäurederivate, deren Synthese und Reaktionen sind dem Fachmann gut bekannt. Für die Kupplungsreaktion, d.h. die Reaktion des N-Terminus einer Aminosäure/ eines Aminosäurederivats mit dem C-Terminus einer anderen Aminosäure/ eines anderen Aminosäurederivats, muss die Aminogruppe der Aminosäure/ des Aminosäurederivats (d.h. an der N_{α}-Position) geschützt werden, da sonst noch die Aminosäure/ das Aminosäurederivat ggf. mit sich selber reagiert. Nach der Kupplungsreaktion kann diese Schutzgruppe vorzugsweise schnell und unter milden Bedingungen abspaltbar sein, damit eine weitere Kupplung erfolgen kann. Die Synthese von Peptiden wird vom C- zum N-Terminus durchgeführt. Als temporäre α-Aminoschutzgruppe sind zwei Urethan-Schutzgruppen gebräuchlich: tert-Butoxycarbonyl (Boc), das mit Protonen, d.h. in saurer Umgebung, abgespalten werden kann, und Fluorenylmethoxycarbonyl (Fmoc), das durch Reaktion mit sekundären Aminen abgespalten werden kann. Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. P. G. M. Wuts, T. W. Greene, Greene's Protective Groups in Organic Synthesis, 4. Auflage, Juni 2006, Wiley) zusätzlich geschützt, wobei in erster Linie Arg(Tos), Arg(Mts), Arg(Mtr), Arg(Pmc), Asp(OBzl), Asp(tert-But), Cys(4-MeBzl), Cys(Acm), Cys(SBut), Glu(OBzl), Glu(OBut), His(Tos), His(Fmoc), His(Dnp), His(Trt, Lys(CI-Z), Lys(Boc), Met(O), Ser(Bzl), Ser(But),Thr(Bzl), Thr(Bzl), Thr(But), Trp(Mts), Trp(CHO), Tyr(Br-Z), Tyr(Bzl) oder Tyr(But) eingesetzt werden können.

Die Peptide können im Rahmen der vorliegenden Erfindung unter Berücksichtigung allgemein bekannten Verfahren der Peptidchemie hergestellt werden, siehe z. B. Houben-Weyl, Methoden der organischen Chemie, Band 15/2, ebenso wie B. Merrifield, J. Am. Chem. Soc. 85,2149 (1963) oder R.C.Sheppard, Int. J. Peptide Protein Res. 21,118 (1983).

Eine "Schutzgruppe", wie hier verwendet, betrifft somit ein Molekül, das mit einer oder mehreren bestimmten Funktionalitäten eines ersten Moleküls oder eines zweiten Moleküls derart reagiert, dass diese Funktionalität für eine andere Reaktion, gegenüber der Verlängerung/ Elongation des Oligomers unempfindlich ist. Durch geeignete Wahl von Reaktionsparametern kann die Loslösung der Schutzgruppe unter geeigneten Bedingungen erfolgen. Vorzugsweise handelt es sich bei dem detektierbaren Marker gleichzeitig um eine Schutzgruppe, wodurch die Synthese des Oligomers vereinfacht wird und zusätzliche Reaktionsschritte vermieden werden können. Die Schutzgruppenchemie zum Aufbau verschiedener Oligomere bzw. zur gezielten Reaktion bzw. Reaktionsunterbindung von Seitenkettenfunktionalitäten ist dem Fachmann gut bekannt.

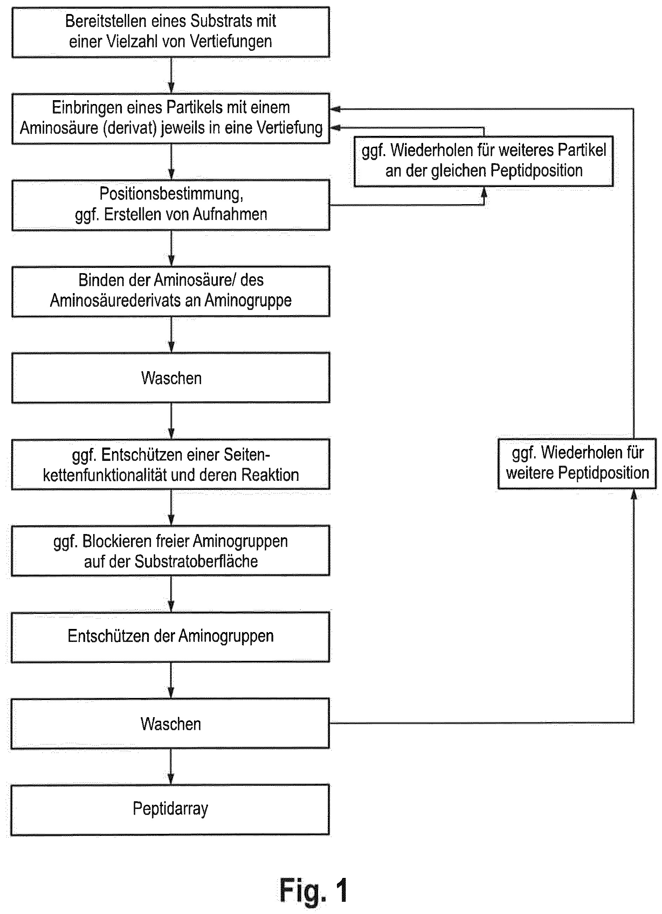

Eine Möglichkeit zur Synthese eines erfindungsgemäßen Peptidarrays wird in der Fig. 1 aufgezeigt. Hierfür wird ein Substrat mit einer Vielzahl von Vertiefungen (mehr als 10⁶ Spots/cm²) bereitgestellt. Ein Monomerpartikel, d.h. ein (einzelnes) Partikel mit jeweils einer Aminosäure bzw. einem Aminosäurederivat wird in eine Vertiefung verbracht. Vorzugsweise ist das Monomerpartikel gemäß der darin enthaltenen Aminosäure/ Aminosäurederivat mit einem detektierbaren Marker derart versehen, dass durch eine Aufnahme, vorzugsweise mittels einer Fluoreszenzaufnahme, die Identität der Aminosäure bzw. des Aminosäurederivats der Vertiefung und damit der Position auf dem Substrat zugeordnet werden kann. Dies wird vorzugsweise für ein oder mehrere Monomerpartikel mit (jeweils) einer anderen Aminosäure bzw. Aminosäurederivat wiederholt. Die Aminosäure bzw. das Aminosäurederivat wird an bereits vorhandene, auf der Substratoberfläche immobilisiert vorliegende Amin-Gruppen gebunden. Die Amin-Gruppen können einerseits den N-Terminus einer bereits immobilisierten Aminosäure bzw. eines Peptids oder eine Oberflächenfunktionalität darstellen, die unmittelbar bzw. mittelbar über einen Spacer auf der Substratoberfläche in einer Vertiefung vorliegt. Durch Waschen werden die Polymermatrix des Monomerpartikels, ungebundene Aminosäuren und andere Verunreinigungen entfernt. Seitenkettenfunktionalitäten der Aminosäuren bzw. der Aminosäurederivate können entschützt werden und miteinander oder mit weiteren Substanzen, beispielsweise Aminosäure bzw. Aminosäurederivaten, umgesetzt werden. Sofern eine erste Lage von Aminosäuren bzw. Aminosäurederivaten auf das Substrat aufgebracht wurde, werden die freien Aminogruppen auf der Substratoberfläche blockiert. Das Substrat wird erneut gewaschen. Im Anschluss erfolgt die erneute Ablagerung von Monomerpartikeln zum Ausbilden einer weiteren Peptidposition bis das Peptid die gewünschte Länge erreicht.

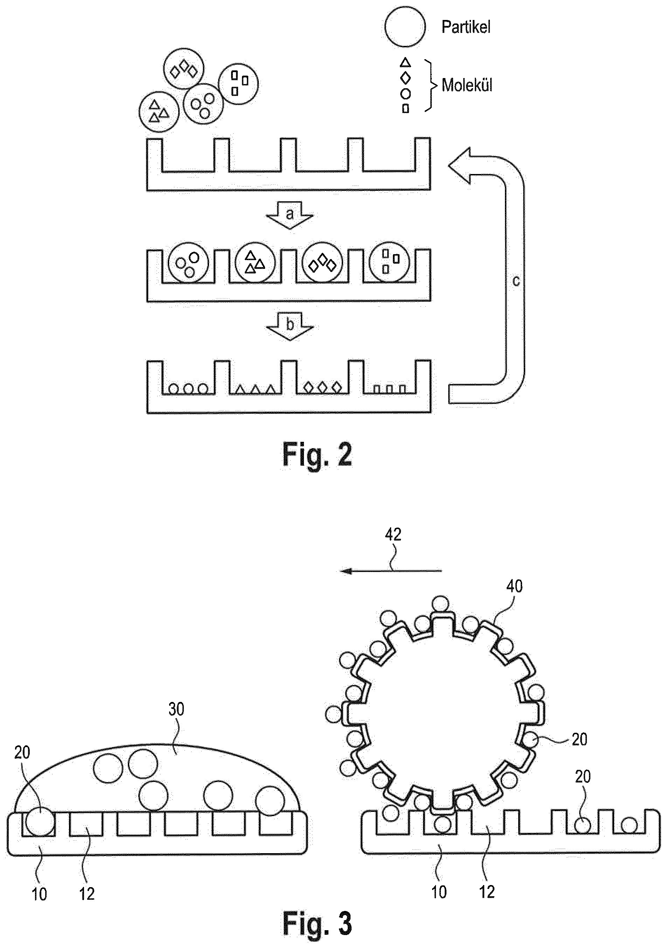

Fig. 2 kann die Ablagerung die Ablagerung von Monomerpartikeln mit jeweils einem darin befindlichen Molekül entnommen werden. Schritt a zeigt die zufällige Ablagerung der jeweiligen Partikel in jeweils eine Vertiefung eines Substrats. Schritt b zeigt die Immobilisierung der Moleküle an der Substratoberfläche. Es ist zu erkennen, dass die Verwendung von Partikeln, und insbesondere von Monomerpartikeln, das eindeutige Verbringen eines Moleküls, in eine bestimmte Vertiefung ermöglicht und es somit nicht nur erlaubt, die Identität des Moleküls, sondern auch dessen Position auf dem Array, eindeutig zu bestimmen. Die vorliegenden Schritte werden für jede Moleküllage (Schritt c) wiederholt, bis der Polymerarray die erwünschte Länge erreicht hat.

Die Ablagerung der Partikel erfolgt vorzugsweise stochastisch. Die Fig. 3 zeigt zwei Möglichkeiten, wie die Partikel in die Vertiefung 12 eines Substrats verbracht werden können. Die Partikel sind hierbei vorzugsweise Monomerpartikel, d.h. ein Partikel umfasst lediglich ein bestimmtes Molekül, beispielsweise Glycin. Derartige Monomerpartikel sind bevorzugt, da andere Monomerpartikel, d.h. Partikel mit einem anderen Monomer, beispielsweise Biotin, für gewöhnlich nacheinander aufgebracht werden, wodurch das Bestimmen der Art des Monomers und dessen Position auf dem Array vereinfacht wird. Außerdem kann hierbei die Zahl an detektierbaren Markern verringert werden. Alternativ können Mischungen von Monomerpartikeln eingesetzt werden, beispielsweise ein erstes Partikel mit einem ersten Molekül, beispielsweise Glycin, und ein anderes erstes Partikel mit einem zweiten Molekül, das von dem ersten Molekül verschieden ist, beispielsweise Biotin. Eine weitere Möglichkeit besteht darin, Partikel einzusetzen, die mehrere verschiedene Moleküle umfassen.

In der linken Abbildung sind die Partikel 20 in einer Flüssigkeit 30, beispielsweise Wasser oder einem Lösungsmittel, suspendiert. Nach Aufbringen der Suspension können die Partikel beispielsweise in die Vertiefungen 12 mit einem Tuch oder Wischer eingerieben werden. Wie in der rechten Abbildung gezeigt, können die Partikel 20 wahlweise mit einer Walze 40 in die Vertiefungen 12 des Substrats verbracht werden. Die Walze 40 weist hierbei eine mikrostrukturierte Oberfläche mit Abmessungen auf, die den Abmessungen einer Vertiefung des Substrats im Wesentlichen entsprechen. Die mikrostrukturierte Oberfläche weist beispielsweise Erhebungen, Vertiefungen und Abstände dazwischen auf, mit einer Abmessung, die der Partikelgröße entspricht oder einer größeren Abmessung, beispielsweise 10% größerer Abmessungen oder mehr, wie 25% größerer Abmessungen oder mehr, 50% größerer Abmessungen oder mehr, 100% größerer Abmessungen oder mehr, oder 500% größerer Abmessungen oder mehr. Durch Drehen bzw. Rollen 42 der Walze können die Partikel 20 in die Vertiefungen 12 verbracht werden.

In einer Ausführungsform weist das erste Molekül des ersten Partikels eine Aminogruppe mit einer Aminoschutzgruppe und eine freie Carboxygruppe auf.

In einer Ausführungsform wird nach Schritt (d) die Aminoschutzgruppe entfernt.

Nach Schritt (b), (c) oder (d) wird eine Bestimmung des detektierbaren Markers in Abhängigkeit einer Position der Vertiefung auf dem Oligomerarray vorgenommen.

Der detektierbare Marker wird vorzugsweise nach dem Schritt (b) bestimmt. In diesem Fall liegt der detektierbare Marker vorzugsweise auf dem und/oder in dem Partikel vor. Mehr bevorzugt liegt der der detektierbare Marker ausschließlich in dem Partikel eingebettet vor.

Falls die Bestimmung des detektierbaren Markers nach Schritt (c) und/oder (d) erfolgt, ist der der detektierbare Marker für gewöhnlich an das erste Molekül kovalent gebunden. Der Marker fungiert hierbei vorzugsweise gleichzeitig als Schutzgruppe. Es ist klar, dass der detektierbare Marker vor einer gegebenenfalls weiter zu erfolgenden Verlängerung des Oligomers entfernt werden muss, um die Detektion des ersten Moleküls im nachfolgenden Schritt nicht zu beeinträchtigen.

Wie bereits vorstehend genannt, können mehrere detektierbare Marker, wie beispielsweise 2, 3, 4, 5, 6, 7, 8, 9, 10 oder mehr, verwendet werden. Diese dektierbaren Marker können ebenfalls in verschiedenen Konzentrationen verwendet werden. Ferner kann eine Kombination von detektierbaren Markern auf der Oberfläche des Partikels vorliegen, wohingegen das erste Molekül üblicherweise einen, vorzugsweise aber keinen detektierbaren Marker, aufweist, damit die Kupplungsreaktion mit dem zweiten Molekül aus (ggf. sterischen Gründen) nicht beeinträchtigt wird. Allerdings kann es vorteilhaft sein das erste Molekül zu markieren. Hierbei bieten sich vor allem detektierbare Marker mit kleinen räumlichen Abmessungen an, wie beispielsweise radioaktive detektierbare Marker. Radioaktive Marker haben ferner den Vorteil, dass sie in das erste Molekül aufgenommen werden können und somit einen Bestandteil des ersten Moleküls bilden.

Schritt (f) umfasst das Erstellen einer 3D-Ablagerungsmaske aus den Bestimmungen des detektierbaren Markers in Abhängigkeit der Position der Vertiefungen auf dem Oligomerarray.

Die 3D-Ablagerungsmaske kann aus allen Aufnahmen, die zur Bestimmung der detektierbaren Marker vorgenommen wurden, erstellt werden. Dies erfolgt vorzugsweise automatisch, beispielsweise mittels eines Computers und eines geeigneten Computerprogramms. Die 3D-Ablagerungsmaske stellt die Information über die genaue Position eines jeden Oligomers bereit, z.B. wird das Oligomer einer Vertiefung zugeordnet oder anderweitig dessen Position auf dem Substrat mittels beispielsweise eines Koordinatensystems erfasst. Darüberhinaus wird die Abfolge der einzelnen Moleküle des Oligomers und deren Identität erfasst. Ferner können andere Informationen wie beispielsweise ggf. durchgeführte Ringschlüsse und/oder Seitenkettenmodifikationen einzelner Oligomermoleküle in der 3-D Ablagerungsmaske enthalten sein.

In einer Ausführungsform erfolgt nach dem Schritt (d) ein chemisches Modifizieren des immobilisierten Oligomers, wobei ein chemisch modifiziertes Oligomer erhalten wird.

Derartige chemische Modifikationen sind dem Fachmann bekannt und umfassen Ringschlüsse zwischen einzelnen Molekülen eines Oligomers und/oder Seitenkettenmodifikationen. Seitenkettenmodifikationen beinhalten die Reaktion einer Seitenkette eines ersten Moleküls nach erfolgter Kupplungsreaktion mit einem oder mehreren weiteren Molekülen. Auf diese Art können beispielsweise verzweigte Oligomere erzeugt werden oder bestimmte Funktionalitäten in das Oligomer eingefügt werden.

Reaktionspartner, die zur chemischen Modifikation eingesetzt und in ein zu synthetisierendes Oligomer eingebracht werden sollen, werden vorzugsweise ebenfalls mittels eines Partikels in die Vertiefung verbracht. Dies hat den Vorteil, dass ggf. ebenso die chemische Modifikation mittels eines detektierbaren Markers bestimmt werden kann.

In einer Ausführungsform ist der detektierbare Marker ein entfernbarer detektierbarer Marker, der mittels optischer Systeme, wie bspw. Lichtmikroskopie, bestimmt werden kann.

Derartige Marker sind dem Fachmann geläufig. Geeignete detekierbare Marker sind beispielsweise in der WO 2015/066400 beschrieben.

In einer Ausführungsform umfasst das erste Partikel eine Polymermatrix, in die das erste Molekül eingebettet ist.

Die Polymermatrix kann aus einem Polymer bestehen oder aus einem Gemisch mehrerer Polymere. Vorzugsweise handelt es sich um ein Styrol-Acrylat-Copolymer. Die Polymermatrix kann in verschiedenen Formen, vorzugsweise im Wesentlich sphärisch bzw. kugelförmig, mit variablen Abmessungen bzw. Durchmesser erzeugt werden.

Gemäß der Herstellungsverfahren, die dem Fachmann geläufig sind, können Partikel mit variablem Durchmesser erhalten werden. In diesem Fall werden Partikel mit unerwünscht kleinem Durchmesser entfernt. Dies kann beispielsweise durch Zentrifugieren erreicht werden. Zu große Partikel stellen für gewöhnlich kein Problem dar, da diese nicht in die Vertiefung des Substrats passen und in Folge nicht an der Reaktion teilnehmen und beispielsweise durch Anwenden von Druckluft ohne weiteres entfernt werden können.

Wird im Rahmen der vorliegenden Erfindung auf einen Mediandurchmesser Bezug genommen, beinhaltet dies eine Größenverteilung bei der mindestens 90 % oder mehr, vorzugsweise 95 % oder mehr, wie 98 % oder mehr, 99 % oder mehr oder 99,5 % oder mehr, der Partikel den angegebenen Durchmesser aufweisen. Die restlichen Partikel, d.h. die Differenz zu 100%, weist vorzugsweise eine Abweichung vom Mediandurchmesser von ± 50 % oder weniger auf, wie beispielsweise 35% oder weniger oder 10 % oder weniger. Wenn beispielsweise Partikel mit einem Mediandurchmesser von 3 µm beschrieben werden, weisen 90 % oder mehr der Partikel die Größe von 3 µm auf. Die restlichen 10 % oder weniger weisen eine Größenverteilung im Bereich von 1,5 µm bis 4,5 µm auf.

Die Synthese der Polymerpartikel erfolgt vorzugsweise derart, dass das erste Molekül darin aufgenommen ist. Vorzugsweise handelt es sich bei dem ersten Molekül um ein Monomer. Allerdings kann das erste Molekül selbst ein weiteres Oligomer sein, wie beispielsweise ein Dimer, Trimer oder Tetramer, so dass das zu synthetisierende Oligomer um das weitere Oligomer, wie bspw. um das Dimer, Trimer bzw. Quatromer, verlängert wird. Dies hat den Vorteil, dass konservierte Bereiche in den Oligomeren des Oligomerarrays erzeugt werden, d.h. Bereiche, die in allen Oligomeren des Oligomerarrays identisch sind. Das erste Molekül kann ferner mit einer Schutzgruppe und/oder einem detektierbaren Marker versehen sein.

Das Partikel mit dem ersten Molekül, vorzugsweise mit darin eingebettetem ersten Molekül, kann durch einfache Techniken, beispielsweise Wischen, in die Vertiefungen des Substrats eingebracht werden. Durch den Einfluss von beispielsweise Temperatur (Erhitzen) und/oder Chemikalien wird die Polymermatrix durchlässig, d.h. perforiert und/ oder aufgelöst, und das darin enthaltene erste Molekül wird freigesetzt.

Die Verarbeitung geeigneter Polymermatrizen und darin befindlicher Moleküle kann beispielsweise der WO 2014/169928 A1 entnommen werden.

In einer Ausführungsform weist das Verfahren zur Herstellung des Oligomerarrays die folgenden Schritte auf: a) Bereitstellen eines Substrats mit einer Vielzahl von Vertiefungen; b) stochastisches Einbringen eines ersten Partikels mit einem ersten Molekül in eine Vertiefung; c) Freisetzen des ersten Moleküls von dem ersten Partikel; d) Binden des ersten Moleküls an ein zweites Molekül unter Bildung eines Oligomers, wobei das zweite Molekül in der Vertiefung immobilisiert ist; e) optional Wiederholen der Schritte (b) bis (d) unter Verlängerung des Oligomers; wobei mindestens ein erster Partikel und/oder ein erstes Molekül einen detektierbaren Marker aufweist. Eine Vielzahl erster Partikel liegen hierbei vor. Vorzugsweise umfasst jeder erster Partikel identische erste Moleküle, d.h. in einem bestimmten ersten Partikel liegen identische erste Moleküle vor, wohingegen in einem anderen ersten Partikel erste Moleküle vorliegen können, die von dem ersten Molekül des bestimmten ersten Partikels verschieden sind. Mehr bevorzugt weist jedes erste Partikel einen detektierbaren Marker auf, der das erste Partikel entsprechend dem dazugehörigen ersten Molekül kennzeichnet. Die Abfolge der Schritte b), c) und d) beschreibt somit den Aufbau einer Schicht des Arrays, wohingegen der optionale Schritt e) den Aufbau einer (oder mehrerer) folgenden Schicht beschreibt.

Zwischen den Schritten b) und c) liegen vorzugsweise die Schritte vor: b)i)Bestimmen der Position des ersten Partikels und b)ii) Wiederholen der Schritte b) und b)i) bis im Wesentlichen alle Vertiefungen mit einem ersten Partikel befüllt sind. Das optionale Wiederholen der Schritte (b) bis (d) schließt somit ein Wiederholen b), b)i), b)ii), c) und d) ein. Diese alternative Ausführungsform spiegelt hierbei die vorstehende genannte zweite Variante wieder, bei der auf die Markierung des ersten Partikels und/oder ersten Moleküls verzichtet werden kann. Dies wird dadurch ermöglicht indem in Schritt b) lediglich ein definiertes erstes Partikel mit einem definierten ersten Molekül eingesetzt werden, so dass in Schritt b)i) der künftige Ablagerungsort eines definierten, d.h. bekannten, ersten Moleküls eindeutig erfasst wird. Dies wird in Schritt b)ii) für alle verschiedenen Arten erster Partikel und somit entsprechender erster Moleküle durchgeführt bis im Wesentlichen alle Vertiefungen befüllt sind. Die Abfolge der Schritte b), b)i), b)ii), c) und d) beschreibt somit den Aufbau einer Schicht des Arrays, wohingegen der optionale Schritt e) den Aufbau einer (oder mehrerer) folgenden Schicht beschreibt. Es ist klar, dass ein Befüllen von "im Wesentlichen" aller Vertiefungen mit einem ersten Partikel den gewünschten Füllgrad beschreibt, der gemäß den Bedürfnissen variiert werden kann. So kann es bspw. erwünscht sein, dass nicht alle Vertiefungen befüllt werden und somit in bestimmten Vertiefungen kein Elongationsschritt durchgeführt, wodurch ein Oligomerarray mit verschiedenen Oligomerlängen erhalten werden kann.

In einer Ausführungsform erfolgt in Schritt (b) das Einbringen eines einzelnen ersten Partikels mit einem ersten Molekül in eine einzelne Vertiefung. Mithin weist die Vertiefung einen Querschnitt und eine Tiefe auf, dass ein einzelnes Partikel in eine einzelne Vertiefung passt. In Folge liegt ein einzelnes Partikel in einer einzelnen Vertiefung vor. Dies ermöglicht über die Bestimmung des detektierbaren Markers die eindeutige Zuordnung des Partikels zu einer Vertiefung und damit auch des ersten Moleküls. Vorzugsweise handelt es sich bei dem ersten Molekül um mehrere identische erste Moleküle, wie beispielsweise zwei oder mehrere identische erste Moleküle, mehr bevorzugt um zwei bis acht identische erste Moleküle. Somit liegen in einem bestimmten ersten Partikel mehrere erste identische Moleküle vor, wohingegen in einem anderen ersten Partikel mehrere erste identische Moleküle vorliegen, die von den mehreren ersten identischen Molekülen des bestimmten ersten Partikels verschieden sind. Ein Bestimmen des Passens eines einzelnen Partikels in eine einzelne Vertiefung erfolgt vorzugsweise im Verlauf der Herstellung des erfindungsgemäßen Oligomerarrays, d.h. bei der Bestimmung des detektierbaren Markers.

In einer Ausführungsform erfolgt Schritt (c) und/oder Schritt (d) des erfindungsgemäßen Verfahrens in der Anwesenheit eines Lösungsmittels in der Dampfphase. Mithin erfolgt die Freisetzung des ersten Moleküls von dem ersten Partikel bzw. aus dem ersten Partikel und/oder das Binden des ersten Moleküls in der Anwesenheit eines Lösungsmittels in der Dampfphase. Das Substrat wird hierzu einer ungesättigten und/oder gesättigten Dampfatmosphäre aus dem Lösungsmittel ausgesetzt. Der Dampf kondensiert an der Oberfläche des Substrats und/oder wird von dem ersten Partikel absorbiert. Dadurch wird die Polymermatrix durchlässig, d.h. die Polymermatrix perforiert und/oder wird aufgelöst, und das darin enthaltene erste Molekül wird freigesetzt bzw. die darin enthaltenen ersten Moleküle werden freigesetzt. Im Gegensatz zum alleinigen Freisetzen durch Temperaturerhöhung lassen sich dadurch einerseits deutlich größere Mengen an Molekülen freisetzen und andererseits können diese freigesetzten Moleküle leichter an die reaktiven Gruppen auf dem Substrat diffundieren. Bei dem Lösungsmittel handelt es sich vorzugsweise um ein oder mehrere organische Lösungsmittel, wie beispielsweise Dichlormethan, Aceton, N,N- Dimethylformamid und/oder deren Kombinationen. Das Lösungsmittel wird in die Vertiefung eingebracht und anschließend erwärmt. Vorzugsweise werden die Vertiefungen dem Lösungsmittel-Dampf ausgesetzt. Die Extraktion der Moleküle kann bei Temperaturen zwischen -20°C und +110°C, vorzugsweise 10 bis 80°C, mehr bevorzugt 60 bis 80°C, erfolgen. Höhere Temperaturen von bspw. >80°C bis 110°C erleichtern im Allgemeinen die Extraktion und Bindung der Moleküle. Niedrigere Temperaturen von bspw. -20°C bis <10°C verhindern die Diffusion der Moleküle während der Extraktion. Diese Extraktion kann zwischen 1 Minute und 90 Minuten, vorzugsweise 20 bis 60 Minuten, dauern.

In einer Ausführungsform wird eine Oberflächenfunktionalität auf Stegen zwischen einzelnen Vertiefungen chemisch verändert. Dies erfolgt vorzugsweise im Rahmen der Immobilisierung des zweiten Moleküls, wobei dieses die Oberflächenfunktionalität aufweist. Die Immobilisierung des zweiten Moleküls erfolgt hierbei nicht nur in den Vertiefungen, sondern über das gesamte Substrat. Die derart immobilisierten zweiten Moleküle werden im Anschluss in Abhängigkeit von dem Immobilisierungsort derart modifiziert, dass die physiochemischen Eigenschaften der Stege zwischen den Vertiefungen und der Vertiefungen selber variieren. Die Oberfläche der Stege zwischen den Kavitäten kann derart modifiziert werden, dass sie die Diffusion und damit das Binden des ersten Moleküls aus einer Kavität heraus zu den benachbarten Kavitäten verhindert.

Beispielsweise kann diese Modifikation der Oberfläche zwischen den Stegen durch Ätzen der Oberfläche der Stege geschehen, wobei die Vertiefungen selbst nicht dem Ätzmedium ausgesetzt werden. Die Vertiefungen können einerseits beispielsweise durch eine Polymermatrix, welche vor dem Ätzprozess in die Kavitäten eingebracht und zwecks einer homogenen Füllung gesintert wird, vor dem Ätzmedium geschützt werden. Alternativ kann die Oberfläche in den Vertiefungen durch eine aufgesputterte Metallschicht vor dem Ätzmedium geschützt werden. Hierzu wird das gesamte Substrat mit einer Metallschicht belegt, welche anschließend von der Oberfläche der Stege entfernt wird.

Das Ätzen kann beispielsweise durch ein Reinigungsplasma (Sauerstoff-, Stickstoff- oder Argonplasma oder andere Gasplasmen) oder mithilfe eines flüssigen Mediums einer starken Säure oder Base erfolgen. Einzige Voraussetzung ist, dass das die Kavitäten schützende Material chemisch inert gegen das verwendete Ätzmedium ist. Nachdem das Ätzen der Oberfläche der Stege beendet ist, wird das die Vertiefungen schützende Material aus den Vertiefungen entfernt. Die beispielsweise verwendete Polymermatrix kann hierbei durch geeignete organische Lösungsmittel, die dem Fachmann geläufig sind, entfernt werden. Eine Metallschicht kann durch anorganische Lösungsmittel, die dem Fachmann geläufig sind, entfernt werden.

Nachdem das Ätzen der Oberfläche der Stege zwischen den Kavitäten erfolgt ist, kann die Oberfläche der Stege chemisch modifiziert werden. Beispielsweise können hydrophobe Silane an die beim Ätzprozess entstandenen OH-Gruppen gekuppelt werden. Dadurch kann die Diffusion der Moleküle zwischen den einzelnen Kavitäten während der Extraktion verhindert werden. Neben der Möglichkeit des Ätzens besteht die Möglichkeit, die Oberfläche der Stege durch die Aktivierung von fotosensitiven Gruppen zu modifizieren. Hierzu wird auf die Oberflächenfunktionalität des gesamten Substrats ein fotosensitiver Linker gekuppelt, welcher durch Belichten mit UV-Licht abgespalten werden kann. Diese Belichtung kann beispielsweise mithilfe eines sog. Mask-Aligners, wie er in der Halbleiterfertigung bekannt ist, erfolgen. Nach dem Abspalten des Linkers auf den Stegen zwischen den Kavitäten kann diese Oberfläche hydrophobiert werden, wodurch die Diffusion der Moleküle zwischen den einzelnen Vertiefungen während der später stattfindenden Extraktion verhindert wird. Im Anschluss an die Hydrophobierung wird der fotosensitive Linker in den Kavitäten abgespalten. Im Anschluss kann Schritt (b) Einbringen eines ersten Partikels mit einem ersten Molekül in eine Vertiefung erfolgen und die weiteren Schritte durchgeführt werden.

In einer Ausführungsform wird mindestens eine Vertiefung während Schritt (c) und/oder Schritt (d) des erfindungsgemäßen Verfahrens abgedichtet. Mithin werden während des Schritts des Freisetzens des ersten Moleküls von dem ersten Partikel und/oder während des Schritts des Bindens des ersten Moleküls an ein zweites Molekül unter Bildung eines Oligomers die Vertiefungen abgedichtet. Dies verhindert die Diffusion des ersten Moleküls bzw. der ersten Moleküle aus den Vertiefungen und eine damit einhergehende Kontamination anderer Vertiefungen bzw. eine geringere Umsatzrate. Das Abdichten kann beispielsweise durch das Aufbringen eines Dichtmaterials auf die Substratoberfläche erreicht werden. Die Dichtung kann durch den Kontakt des Dichtmaterials und der Substratoberfläche sichergestellt werden. Folglich stellt jede Kavität ein abgeschlossenes System dar, in der die Freisetzung und/oder das Binden des Moleküls bzw. der Moleküle vollkommen unabhängig von den anderen Kavitäten des Substrats stattfinden. Als Dichtmaterial kommen beispielsweise PDMS, PTFE, PFDV-Membranen oder andere kommerziell erhältliche Klebebänder in Frage. Alternativ oder zusätzlich kann jeder andere Verfahrensschritt, bei dem eine Isolierung von der Umgebung gewünscht ist, entsprechend, d.h. unter Abdichtung der Vertiefung, durchgeführt werden. Ebenso kann die Lagerung des Oligomerarrays dadurch erfolgen, dass vorzugsweise alle Vertiefungen abgedichtet werden.

In einer Ausführungsform wird ein zweites Molekül in der Vertiefung immobilisiert, wobei das zweite Molekül ein abspaltbare Abstandshalter ist. Dadurch können abspaltbare Abstandshalter in die Synthese des Oligomerarrays mit einbezogen werden. Die synthetisierte Oligomere können einfach abgespalten und gegebenenfalls auf eine Zieloberfläche oder in eine Vertiefung eines anderen Substrats transferiert werden. Dies ermöglicht ferner eine Aufreinigung der Oligomere, da vorzugsweise lediglich vollständig synthetisierte Oligomere transferiert werden. Die Abbruchprodukte (nicht vollständig synthetisierte Oligomere) hingegen werden vorzugsweise nicht transferiert und können während eines Waschschritts entfernt werden. Vollständige Oligomere sind diejenigen Oligomere, welche eine Kettenlänge entsprechend der Anzahl der durchlaufenden Durchgänge gemäß dem vorliegenden Verfahren aufweisen, d.h. der Zahl der Monomere des zu erzielenden Oligomers. Zudem können die Oligomerarrays durch den Transfer vervielfältigt werden. Durch Steuern der prozentualen Abspaltrate können somit mehrere Replikate desselben Oligomerarrays erreicht werden.

Die Abspaltung der Moleküle wird durch dem Einbau abspaltbarer Abstandshalter an der Basis der Oligomere erreicht. Solche durch beispielsweise Licht, insbesondere UV-Licht oder Hydrogenolyse oder Photolyse oder unter basischen Bedingungen usw. sowie gegenüber den Reaktionsbedingungen einer Synthese stabilen abspaltbaren Abstandshalter sind dem Fachmann bekannt. Selektiv spaltbare Linker, die auf einer Methionin- und einer Estergruppe basieren können bspw. der DE 69435011 T2 entnommen werden. Fields GB und RL Noble, 1990, Solid phase peptide synthesis utilizing 9-fluorenylmethoxycarbonyl amino acids, Int. J. Pept. Protein Res. 35: 161-214 offenbart weitere derartige Linker. Beispiele weiterer Abstandshalter sind der Rink Amide Linker oder UV spaltbarer Linker, die M.S. Bernatowicz, S.B. Daniels, H. Köster, Tetrahedron Lett. 30 (1989) 4645 bzw. Stefan Peukert and Bernd Giese, The Pivaloylglycol Anchor Group: A New Platform for a Photolabile Linker in Solid-Phase Synthesis, J. Org. Chem. 1998, 63, 9045-9051 entnommen werden können.

In einer Ausführungsform werden die synthetisieren Oligomere, vorzugsweise die vollständig synthetisieren Oligomere, auf eine Zieloberfläche transferiert. Wie vorstehend aufgeführt sind vollständige Oligomere diejenigen Oligomere, welche eine Kettenlänge entsprechend der Anzahl der durchlaufenden Durchgänge gemäß dem vorliegenden Verfahren aufweisen, d.h. der Zahl der Monomere des zu erzielenden Oligomers. Die Zieloberfläche kann eine Vertiefung eines anderen, zweiten Substrats sein, eine plane Oberfläche, bspw. eines Objektträgers, eine Membran, oder irgendein anderes Behältnis sein. Für den Transfer werden die Kavitäten mit einer Pufferlösung befüllt. Die Zusammensetzung des Puffers hängt von der Beschaffenheit der synthetisierten Oligomeren ab und ist dem Fachmann geläufig. Um die beim Transfer auftretende Diffusion der Oligomere aus einer Kavität heraus in benachbarte Kavitäten zu verhindern, kann als Zieloberfläche beispielsweise eine Membran genutzt werden, welche die einzelnen Kavitäten abdichtet. Nach dem Platzieren der Membran erfolgt die Abspaltung des Abstandshalters. Sämtliche Oligomere (komplett synthetisierte Oligomere sowie Abbauprodukte) können sich nun in den Grenzen der Diffusion frei in der mit der Pufferlösung befüllten Kavitäten bewegen.

Um vorzugsweise nur die komplett synthetisierten Oligomere zu transferieren und damit eine Aufreinigung des Oligomerarrays zu erzielen, wird in einem letzten Schritt der Oligomersynthese eine funktionelle Gruppe an die endständigen Gruppen der Oligomere gekuppelt. Diese kann bspw. entsprechend der Festphasensynthese nach Merrifield nur an komplett synthetisierte Oligomere kuppeln, da die Abbauprodukte bereits acetyliert (blockiert) worden sind. Diese funktionellen Gruppen können mit der Zieloberfläche, welche ebenfalls funktionalisiert ist, eine Bindung eingehen. Beispiele dieser Bindungen sind Biotin-Streptavidin, Azid-Alkin oder Thiol-Gold Wechselwirkungen. Nach der Abspaltung der Syntheseprodukte werden nur komplett synthetisierte Oligomere die Bindung mit der funktionalisierten Zieloberfläche eingehen, während die Abbauprodukte weggewaschen werden. Dem Fachmann sind geeignete funktionelle Gruppen geläufig.

In einer Ausführungsform werden die synthetisieren Oligomere, vorzugsweise die vollständig synthetisierten Oligomere, mit einer Probe in Kontakt gebracht. Dies eröffnet die Möglichkeit den fertig synthetisierten Oligomerarray unmittelbar zur Untersuchung der Probe mit den einzelnen Oligomeren zu Unterziehen. Dadurch können stochastische Assays mit den erfindungsgemäßen stochastischen Oligomerarrays durchgeführt werden. Beipsielsweise kann mithilfe der stochastischen Assays die Wirkung von Peptiden auf Baktierien oder Proteine untersucht werden. Bei der Probe kann es sich um jegliche Art von Probe handeln, vorzugsweise handelt es sich um eine flüssige Probe, mehr bevorzugt um Körperflüssigkeiten enthaltene Proben, wie bspw. Blut, Serum oder Urin enthaltende Proben. Die Proben können unmittelbar oder in verdünnter Form eingesetzt werden. Der Assay kann unmittelbar in den Vertiefungen erfolgen.

Um vorzugsweise die Wechselwirkung der vollständig synthetisieren Oligomere mit der Probe zu erleichtern bzw. ggf. überhaupt erst zu ermöglichen, kann ein abspaltbarer Linker zwischen dem Oligomer und der Vertiefung vorliegen, d.h. bei dem zweiten Molekül handelt es sich um den abspaltbaren Linker. Die Vertiefungen werden mit der Probe befüllt. Ein Überschuss an Lösung kann entfernt werden, so dass aufgrund der Oberflächenspannung, von bspw. wässrigenLösungen, in der Vertiefung ein in Richtung der Öffnung der Vertiefun gewölbtes Flüssigkeitsreservoir (konkaver oder konvexer Meniskus) ausgebildet wird. Wird nun ein Deckplättchen, beispielsweise aus Glas, in einem definierten Abstand über dem Substrat positioniert, so gehen die Flüssigkeitsreservoire eine Verbindung mit dem Deckplättchen ein. Anschließend können die abspaltbaren Linker durch beispielsweise UV-Licht abgespalten werden und die Wirkung der abgespaltenen Oligomere auf die Baktierien, die Proteine oder den eingebrachten Stoff untersucht werden. Das Deckplättchen erfüllt hierbei zwei Funktionen. Zum Einen wird durch die plane Oberfläche eine Detektion der zu untersuchenden Wechselwirkungen ermöglicht. Zum anderen verringert das Deckplättchen durch seinen geringen Abstand zum Substrat eine Verdunstung der eingebrachten Lösung.

In einer Ausführungsform ist der Oligomerarray ein fokussierter Oligomerarray.

Ein derartiger fokussierter Oligomerarray enthält konservierte Bereiche, d.h. ein oder mehrere Monomer Moleküle, die in allen Oligomeren des Oligomerarrays identisch vorliegen. Es hat sich gezeigt, dass das erfindungsgemäße Verfahren v.a. für die Erzeugung eines derartigen fokussierten Oligomerarrays geeignet ist.

In einer Ausführungsform umfasst der Oligomerarray eine 3D-Ablagerungsmaske, die eine Zuordnung des mindestens einen ersten Moleküls und des zweiten Moleküls an die 3D-Ablagerungsmaske zu einer Position der Vertiefung auf dem Oligomerarray ermöglicht.

In einer Ausführungsform umfasst ein (endständiges) erstes Molekül einen detektierbaren Marker.

Ein detektierbarer Marker kann jegliche Art von Marker betreffen, beispielsweise Lumineszenzmarker, Fluoreszenzmarker, mittels Hybridisierung detektierbare Marker aber auch radioaktive Marker. Beispiele für geeignete radioaktive detektierbare Marker umfassen ¹¹C, ⁴⁰K, ¹³N, ¹⁵O, ¹⁸F, ⁷⁵Br ⁷⁶Br, ⁸²Rb, ⁶⁸Ga, ⁶⁴Cu, ⁶²Cu, ¹²³I, ¹²⁴I, ¹²⁵I, ¹³¹I, ²¹⁰At, ²¹¹At und ¹¹¹In sind aber nicht darauf begrenzt. Weitere Beispiele für detektierbare Marker betreffen jegliches Atom oder Molekül, das zur Bereitstellung einer nachweisbaren, vorzugsweise qualifizierbaren, Wirkung beiträgt und welches an einem Partikel und/oder ersten Molekül angebracht werden kann. Eine nicht begrenzte Aufführung dieser Marker umfasst beispielsweise Enzyme, die ein nachweisbares Signal erzeugen, beispielsweise durch Kolorimetrie, Fluoreszenz oder Lumineszenz. Beispiele derartiger Enzyme umfassen die Meerrettichperoxidase, alkalische Phosphatase, (Beta)-Galactosidase oder Glucose-6-phosphat-dehydrogenase, Chromophore, wie beispielsweise fluoreszente, lumineszente oder gewöhnliche Farbstoffverbindungen, Gruppen mit einer Elektronendichte, welche durch Elektronenmikroskopie oder über deren elektrischen Eigenschaften, wie beispielsweise Konduktivität, Amperometrie, Voltametrie oder Impedanz nachweisbar sind. Vorzugsweise handelt es sich bei dem detektierbaren Marker um einen Lumineszenzmarker und/ oder Fluoreszenzmarker. Weiter bevorzugt ist der detektierbare Marker ein Fluoreszenzmarker.

Somit umfassen die Oligomere des Oligomerarrays jeweils in der endständigen Position jeweils einen detektierbaren Marker. Die Anwesenheit der detektierbaren Marker kann für die Untersuchung der Oligomere bzgl. deren Bindeverhalten an einen Bindepartner von Nutzen sein. Beispielsweise kann durch die erfolgte Bindung eines Bindepartners an ein bestimmtes Oligomer das Signal dessen detektierbaren Markers (teilweise) ausgelöscht werden, wodurch der Nachweis des Bindeereignisses ermöglicht wird.

In einer Ausführungsform passt lediglich ein Partikel in eine Vertiefung. Somit kann sichergestellt werden, dass lediglich der Molekültyp, die in dem Partikel enthalten sind, zur Reaktion gelangen. Mehr bevorzugt handelt es sich um bei dem Molekültyp um ein definiertes Molekül und nicht um ein Gemisch verschiedener Moleküle.

In einer Ausführungsform wird der erfindungsgemäße Oligomerarray zur Bestimmung eines Bindepartners verwendet.

Der Bindepartner ist vorzugsweise ein medizinisch oder diagnostisch verwertbares Molekül.

DNA/RNA-Stränge können verwendet werden, um ihre(n) komplementäre(n) Strang bzw. Stränge zu binden, und kleine Moleküle können verwendet werden, um Antikörper zu binden. Andere nichteinschränkende Beispiele umfassen Enzyme, Antikörper, konjugierte Enzyme, konjugierte Antikörper, Glykoproteine, Desoxyribonucleinsäuremoleküle, Desoxyribonukleinsäurefragmente (Oligomere), Polymermoleküle, Ribonukleinsäuren, Ribonukleinsäurefragmente, Pharmazeutika, Aptamere, Hormone und/oder Kombinationen derselben.

In einer Ausführungsform wird ein Partikel mit einem Molekül bereitgestellt, wobei mindestens eines des Partikels und/oder des Moleküls einen detektierbaren Marker aufweist. Das erste Molekül liegt hierbei in dem ersten Partikel vor.

Die in der vorliegenden Anmeldung beschriebenen stochastischen Oligomerarrays unterscheiden sich von den bisher herstellbaren Oligomerarrays und insbesondere Peptidarrays zunächst einmal in der Zahl der unterschiedlichen Oligomere, die dadurch im Arrayformat synthetisiert werden können. Bei einem Pitch von 2 µm (25 Mio. Spots/cm²) und bei einer Größe des Objektträgers von ca. 15 cm² ergeben sich mit dem vorliegenden Verfahren etwa 1 Milliarde Peptidspots. Derart große Peptidvariationen, die mit den erfindungsgemäßen stochastischen Oligomerarrays erreicht werden können, wurden bisher nur mit Phagen-Display Verfahren erreicht, so dass diese für typische Hochdurchsatz Durchmusterungsverfahren eingesetzt werden können, insbesondere wenn dafür keinerlei Vorinformation über das zu untersuchende Molekül zur Verfügung steht (z.B. die Suche nach Bindepartner an Oligomere). Ein großer technischer Vorteil der Erfindung besteht in der erheblichen Erhöhung der Spotdichte und der daraus resultierenden sehr hohen Spotanzahl auf einem Träger. Da die Kosten der synthetisierten Arrays im Wesentlichen von der Fläche abhängen, auf der sie synthetisiert werden, ergibt dies - verglichen mit anderen Syntheseverfahren - einen sehr großen Kostenvorteil pro synthetisiertem Oligomer und insbesondere Peptid. Eine mikro- oder gar nanometergenaue Positionierungstechnik wird nicht benötigt, während die anderen Verfahren gemäß dem Stand der Technik kostenintensive, wartungsintensive und defektanfällige Vorrichtungen, wie beispielsweise Lithographiemasken, Drucker und ähnliches, benötigen. Ein weiterer Vorteil des Verfahrens besteht darin, dass das vorliegende Verfahren sehr einfach skaliert werden kann. Es ist mit einem lediglich geringzügigen Zusatzaufwand verbunden, wenn statt nur einem Substrat mit einer kleineren Oberfläche eine größere Oberfläche oder gleich mehrere Substrate mit Oligomeren versehen werden. Durch Anpassung der Partikelgröße an die Größe der Vertiefungen kann die Spotdichte auf bis zu 100 Millionen Spots/cm² und darüber hinaus erhöht werden, was den Stand der (Array)Technik um 3 bis 5 Größenordnungen übertrifft. Mit den derzeit verfügbaren Fluoreszenzverfahren zum Nachweis des einzelnen Moleküle bzw. Monomere der Oligomere ist es möglich, sogar einzelne Farbstoffmoleküle zu detektieren. Mit den stochastischen Oligomerarrays wird erstmals eine volle Kombinatorik mit Aminosäurebausteinen ermöglicht. Für die Ausschöpfung der vollen Kombinatorik eines 5-mer Peptids benötigt man 3,2 Millionen Spots, bei einem 6-mer 64 Millionen Spots und bei 7-mer Peptiden 1,28 Milliarden Spots. Ein weiterer Vorteil - im Vergleich zu den bioxerografischen Verfahren gemäß dem Stand der Technik - liegt in der vereinfachten Zusammensetzung der Partikel. Die Partikel müssen keine elektrische Ladung aufweisen bzw. ladungsgenerierende und -stabilisierende Additive beinhalten, da man nicht auf die Manipulation der Partikel mit elektrischen Feldern angewiesen ist.

Durch das erfindungsgemäße Verfahren können sogenannte fokussierte Bibliotheken besonders einfach hergestellt werden. Wenn beispielsweise ein durch Hitze schaltbarer Peptidbinder an einenm Antikörper oder an einem Zielprotein gesucht wird, dann können die Aminosäurepositionen, die für die Bindung an den Antikörper oder an das Zielprotein verantwortlich sind, konstant gehalten werden, während die Positionen, die für die Bindung eher unwichtig sind, variiert bzw. permutiert werden können. Anschließend kann die Bindung des Zielproteins an die jeweiligen Peptidvarianten bei verschiedenen Temperaturen überprüft werden, ob denn dadurch ggf. eine Entropie-getriebene und dadurch Temperatur abhängige Variante des ursprünglich gefundenen Peptids auffindbar ist.

Durch das erfindungsgemäße Verfahren können in die Oligomere, insbesondere in die Peptide, alle Arten von Sonderbausteinen eingebaut werden. Dies kann entweder direktwährend der Peptidsynthese passieren, oder auch erst nach erfolgter Synthese z.B. durch eine Klickreaktion. Letzteres ist insbesondere dann wichtig, wenn der Sonderbaustein die bei der Synthese vorherrschenden Bedingungen (üblicherweise 50% TFA beim Abspalten der Peptide; 20% Piperidin in DMF) nicht aushält, d.h. der Sonderbaustein sich zersetzt bzw. eine Reaktion eingeht. Die Sonderbausteine können alle Arten von Funktionen mitbringen. Porphyrinderivate und Ferrocene können Licht ernten oder Elektronen weiterleiten. Andere Sonderbausteine können magnetische Eigenschaften besitzen oder durch Einstrahlen von Licht ihre Molekülstruktur ggf. reversibel ändern. Wieder andere Bausteine können z.B. über Alkin- und Azidfunktionen nach Zugabe eines Katalysators eine Zirkularisierung der Peptide bewirken.

Viele Antikörper erkennen kurze Fragmente aus 5-15 Aminosäuren, wobei 3 bis 7 der Aminosäuren innerhalb des Peptids für die Spezifität der Bindung verantwortlich sind. Dies bedeutet, dass die Spezifität der Bindung an das (lineare) Antigen bei nahezu jedem monoklonalen Antikörper sehr einfach bestimmt werden kann, wenn mit diesem nur ausreichend viele zufällig hergestellt Peptide im Arrayformat gefärbt werden, ohne dass dafür irgendeine Vorinformation nötig wäre. In einem nachgelagerten Versuch können dann anschließend einige der so gefundenen Peptide an jeder Position systematisch variiert werden, um die sogenannte Bindungssignatur zu bestimmen, d.h. die Aminosäurepositionen, die für die Spezifität der Bindung verantwortlich sind. Mit diesen Informationen kann anschließend in den Datenbanken nach geeigneten Kandidatenantigenen gesucht werden, die ursprünglich diesen Antikörper induziert haben. Das menschliche Proteom lässt sich in Form von etwa 12 Millionen unterschiedlichen Peptiden darstellen. Mit einem stochastischen Peptidarray wird es möglich, das gesamte menschliche Proteom auf einem Objektträger stochastisch abzubilden und als Ganzes in einem Experiment zu erfassen.

Durch das beschriebene erfindungsgemäße Verfahren wird ermöglicht die Bindeepitope bei Antikörpern einfach zu bestimmen, die konformationelle Epitope erkennen. Diese bestehen meist aus zwei Peptid-Schlaufen auf der Oberfläche eines Proteins, die gemeinsam von dem Antikörper erkannt werden. Lineare Peptide können diesen "Schlaufen-Charakter" nicht nachbilden, da diese sehr viele Faltungsmöglichkeiten haben, was auf Kosten der Bindungsaffinität geht. Durch die Herstellung einer großen Zahl unterschiedlicher zirkulärer Peptide ist es möglich, ein zirkuläres Peptid aufzufinden, das eine solche Schlaufe nachbildet. Wie oben beschrieben kann dann anschließend die Bindungssignatur bestimmt werden und in den Datenbanken nach Kandidatenantigenen gesucht werden (insbesondere wenn zwei verschiedene Peptid-Schlaufen zu einem Protein passen).

Ein solches Verfahren kann aber nicht nur zur Charakterisierung monoklonaler Antikörper eingesetzt werden, sondern auch zur Charakterisierung von Antikörpergemischen, wie sie in den Seren von Patienten vorkommen. Die vorliegende Erfindung kann die Bestimmung einiger Dutzend bis mehreren hundert unterschiedliche Signaturen (wie vorstehend beschrieben) pro Patientenserum, ganz ohne Vorinformation, zu bestimmen und anschließend nach Korrelationen zwischen gefundenen Antikörpern und Krankheitszuständen zu suchen.

Ganz analog zur Suche nach Antikörper-charakterisierenden Peptiden, ermöglicht das erfindungsgemäße Verfahren mit zirkularisierten Peptiden auch nach Bindern an therapeutisch interessanten Zielproteinen zu suchen. Dies ist bei linearen Peptiden oftmals unmöglich, da die (zu) vielen Faltungsmöglichkeiten eines linearen Peptids die Bindungsaffinität an ein interessantes Zielprotein häufig nachteilig beeinflussen.

Die Erfindung wird nachstehend anhand von Beispielen dargestellt und in der nachfolgenden Beschreibung näher erläutert.

### BEISPIELE

### Beispiel 1: Einbringen der Partikel in die Vertiefungen eines Substrats.

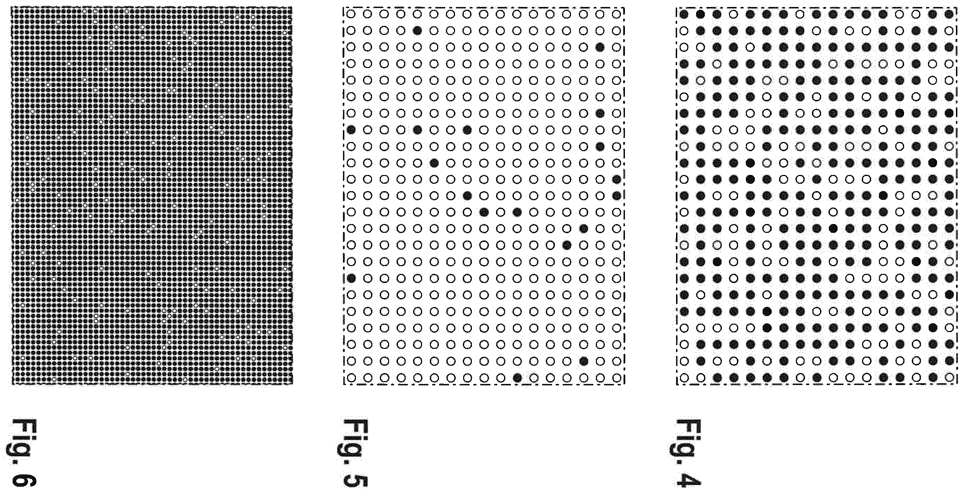

In den Fig. 4 bis Fig. 7 wird das Befüllen von Vertiefungen auf einem Substrat veranschaulicht. Es ist ersichtlich, dass der Füllgrad der Vertiefungen über einen weiten Bereich gemäß den Erfordernissen variiert werden kann und ferner das Befüllen der Vertiefungen stochastisch erfolgt.

Die Fig. 4 zeigt das 60 %ige Befüllen zylindrischer Vertiefungen (Durchmesser 5 µm, Pitch 10 µm, Tiefe 5 µm) mit Polymerpartikeln (Durchmesser 4 µm).

In der Fig. 5 wird ein 5 %iges Befüllen zylindrischer Vertiefungen (Durchmesser 6 µm, Pitch 4 µm, Tiefe 5 µm) mit Partikeln (Durchmesser 4,21 µm) gezeigt. Die Partikel wurden in Suspension in die Vertiefungen eingebracht, die Konzentration der Suspension beträgt 2 µl Partikel in 2,4 mL Wasser.

Die Fig. 6 zeigt 6,4 %iges Befüllen von zylindrischen Vertiefungen (Durchmesser 0,6 µm, Pitch 2 µm, Tiefe 1 µm) mit Glycin-Aminosäurepartikel. Das Partikelmuster wurde mit Hilfe einer Walze hergestellt.

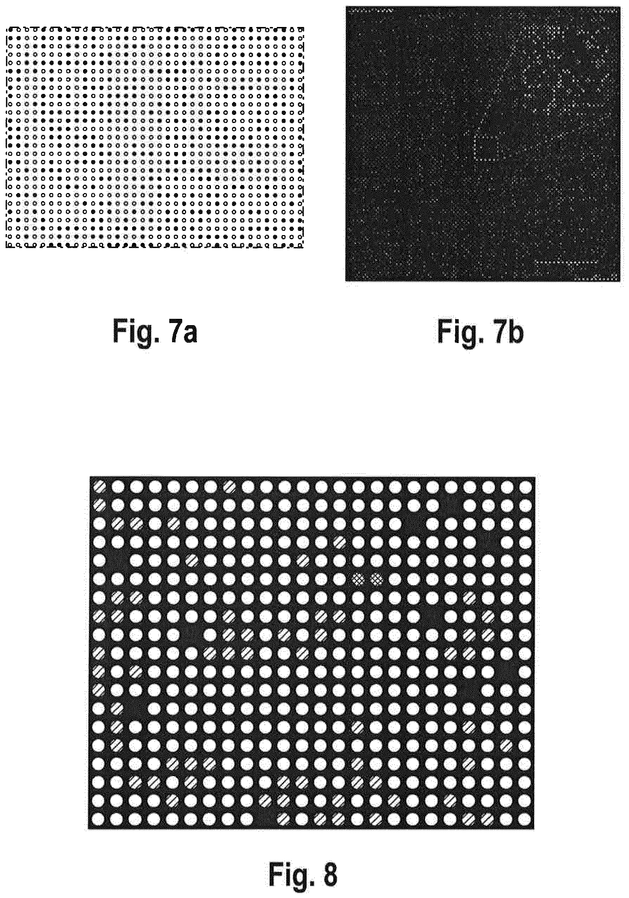

In der Fig. 7 ist ein Beispiel zur Markierung von Monomerpartikeln mit FluoreszenzFarbstoffen gezeigt, Lichtmikroskopaufnahme (Fig. 7a) und Fluoreszenzbild der zufällig abgelagerten Partikel (Fig. 7b). Die Lage der Monomere wird aufgrund der Fluoreszenzfarbe identifiziert (helle Kreise symbolisieren hierbei Rot- und Grünfärbung, wobei die Rotfärbung etwas heller erscheint). Ungefüllte Vertiefungen werden für die Ablagerung nachfolgender Monomere benutzt, Pitch 10 µm.

### Beispiel 2: Herstellung eines Dipeptidarrays aus Glycin- und Biotinmonomeren.

Weitere Details zur Durchführung und die dabei verwendeten Materialien und Methoden können dem nachstehenden Beispiel 3 entnommen werden.

In einem ersten Schritt wird ein geeignetes Substrat hergestellt. Ein Glasobjektträger wird mit Vertiefungen eines Durchmessers von 1 µm und einer Tiefe von 1 µm bereitgestellt. Der Pitch der Vertiefungen beträgt 2 µm. Die die Oberfläche des Substrats wird mit einer Poly(ethylenglycol)methacrylat Graft- bzw. Propf-Polymerschicht (10:90 PEGMA-co-MMA Polymer) versehen und anschließend mit NH₂-Gruppen funktionalisiert, um die Peptidsynthese zu ermöglichen.

Glycin- und OPfp-aktivierte Biotin-Partikel mit einem Mediandurchmesser von 3 µm werden aus einem Styrol-Acrylat-Copolymer als Matrix und entsprechenden Monomeren (Glycinderivat und Biotin) wie in der WO 2014/169928 A1 beschrieben hergestellt. Die Partikel werden mit Hilfe einer elastischen Gummiwalze, wie schematisch links in der Fig. 3 gezeigt, in den Vertiefungen auf dem funktionalisierten Glasträger abgelagert. Die Position der abgelagerten Glycin-Partikel bzw. Biotin-Partikel werden mittels Leitz Ergolux 200 Lichtmikroskop-Aufnahmen bestimmt. Die verwendeten Partikel werden mit Hilfe des Sprühtrocknungsprozess hergestellt und bestehen aus der Polymermatrix und Monomerderivat.

Das Binden an die Amin-funktionalisierte Oberfläche des Substrats erfolgt bei 90° C für 90 Minuten unter Argonatmosphäre in einem vorgeheizten Laborofen (Firma Nabertherm). Hierbei befindet sich das Substrat einer kleinen Metallbox, welche mit Argon gefüllt ist. Die Aminosäurederivate in der Matrix diffundieren in dieser Zeit zum Substrat und binden dort durch Ausbildung einer Amidbindung an die dort funktionalisiert vorliegenden NH₂-Gruppen.

Das Substrat wird einem Waschschritt unterzogen, um die Copolymermatrix, überschüssige Monomere und andere Bestandteile zu entfernen. Zuerst wird die Substratoberfläche lange mit Aceton gespült und in ein Ultraschallbad mit Aceton für 5 Minuten bei einer Frequenz von 132 kHz und einer Leistung von ca. 27 W (30% der effektiven Leistung von 80%) verbracht. Anschließend erfolgt für 5 Minuten dreimaliges Spülen mit DMF und für 3 Minuten zweimaliges Spülen mit MeOH. Die einzelnen Waschschritte werden mit dem Lichtmikroskop kontrolliert.

Freie Aminogruppen auf der Oberfläche des Substrats werden blockiert (acetyliert). Das Blockieren erfolgt über Nacht mit Essigsäureanhydrid-Diisopropylethylamin-Dimetylformamid (ESA-DIPEA-DMF) im Verhältnis von 1:2:7. Anschließend wird drei Mal mit DMF (5 Minuten) und zwei Mal mit MeOH (3 Minuten) gewaschen und das Substrat im Argonstrom getrocknet.

Entschützen der Aminosäuren (Entfernung von Fmoc an den N-Terminalen Enden der Aminosäuren) erfolgt mit Piperidin (20 % v/v) in DMF für 20 Minuten, dreimaligem Waschen mit DMF (für 5 Minuten) und zweimaligem Waschen mit MeOH (für 3 Minuten). Das Substrat wird im Argonstrom getrocknet.

Anschließend werden erneut Glycin-Aminosäurepartikel zur Bildung der zweiten Schicht aufgebracht.

Die Position der abgelagerten Glycin-Partikel wird mit dem Lichtmikroskop bestimmt und Aufnahmen gemacht.

Anschließend erfolgt die Ablagerung von Biotin-Partikeln ebenfalls auf der zweiten Schicht.

Die Position der abgelagerten Biotin-Partikel wird mit dem Lichtmikroskop bestimmt und eine Aufnahme des Substrats erstellt.

Die Kupplungsschritte des Glycinderivats und Biotins erfolgen wie vorstehend beschrieben bei 90° C für 90 Minuten unter Argonatmosphäre.

Das Substrat wird anschließend gewaschen, freie Gruppen werden blockiert, und Fmoc-Gruppen werden zum Entschützen der Aminosäuren entfernt.

Anschließend wird das Substrat mit PBS-T Puffer mit einem pH von 7,4 (ein Liter Millipore Wasser, 500 µL Tween 20, 500 mg NaN₃) für 15 Minuten gewaschen.

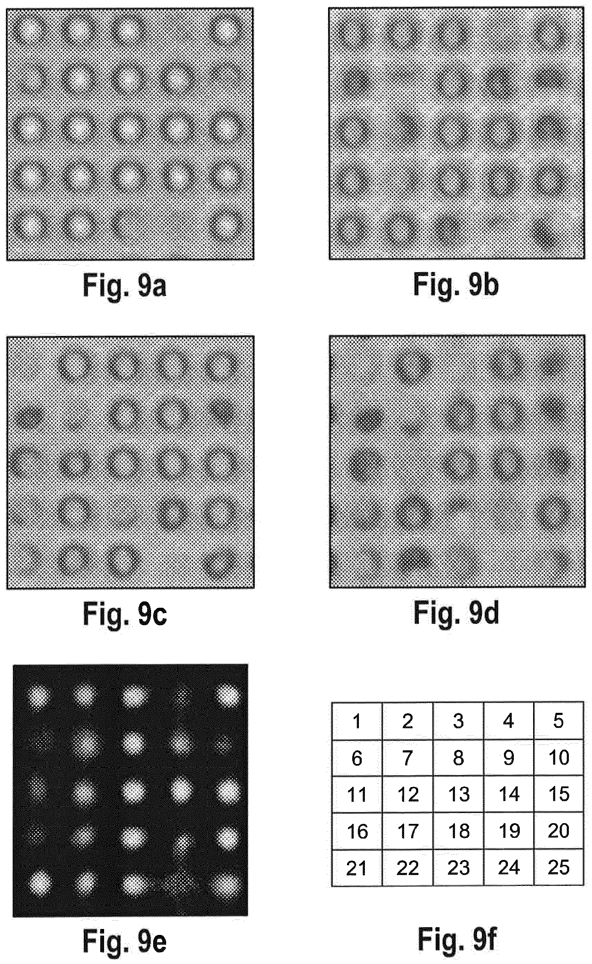

Folgende Kombinationen sind auf dem Substrat zu beobachten: Biotin, Glycin-Glycin, Glycin-Biotin. Zur Kontrolle der Syntheseprodukte wird der Träger anschließend mit Fluoreszenzmarker (NHS-Ester Aktivierung; rot, Anbindung an die freie Aminogruppe von Glycin) und mit fluoreszenzmarkiertem Streptavidin (grün, Anbindung an Biotin) gefärbt. Hierbei weisen die Glycin-Glycin Spots rote Signale und die Spots mit Biotin bzw. Glycin-Biotin grüne Signale auf. Andere Spots, die keine der genannten Varianten tragen, weisen erwartungsgemäß keine Fluoreszenzsignale auf. Eine Fluoreszenzaufnahme des stochastischen Dipeptidarrays ist in Fig. 8 gezeigt. Die Abbildung 9 zeigt beispielhaft 25 Strukturen nach der stochastischen Ablagerung der zwei Monomere in jeder der beiden Lagen. Fig. 9a (Glycin 1) und Fig. 9b (Biotin 1) zeigen den gewählten Ausschnitt nach der stochastischen Ablagerung des jeweiligen Monomers der ersten Lage. Die Fig. 9c (Glycin 2) und Fig. 9d (Biotin 2) die Ablagerungen der zweiten Lage. Ein Fluoreszenzbild nach der Färbung des synthetisierten Arrays wird in der Fig. 9e gezeigt, Um die einzelnen Strukturen zuordnen zu können, werden sie von 1 (oben links) bis 25 (unten rechts) nummeriert (Fig. 9f). Das Fluoreszenzbild nach der Färbung des synthetisierten Arrays ist ebenfalls dargestellt. Die Auswertung der Belegung der einzelnen Lagen sowie die Verifikation der Fluoreszenzaufnahme (Tabelle 1) zeigt die Übereinstimmung der detektierten Belegung mit den erwarteten Fluoreszenzsignalen. Das + symbolisiert hierbei die Detektion des jeweiligen Bausteins in der untersuchten Struktur.

**Tab 1: Dipeptidsynthese**

| Nummer | Fmoc-Glycin-OPfp 1 | Biotin 1 | Fmoc-Glycin-OPfp 2 | Biotin 2 | Farbe (Theorie) | Farbe (Real.) |
|---|---|---|---|---|---|---|
| 1 | | | + | | schwarz | schwarz |
| 2 | | | | | schwarz | schwarz |
| 3 | | | | + | schwarz | schwarz |
| 4 | + | | | | schwarz | schwarz |
| 5 | | | | + | schwarz | schwarz |
| 6 | | | | | schwarz | schwarz |
| 7 | | + | + | | grün | grün |
| 8 | | | | | schwarz | schwarz |
| 9 | | | | | schwarz | schwarz |
| 10 | | | | | schwarz | schwarz |
| 11 | | | | | schwarz | schwarz |
| 12 | | + | | + | grün | grün |
| 13 | | | | | schwarz | schwarz |
| 14 | | | | | schwarz | schwarz |
| 15 | | | | | schwarz | schwarz |
| 16 | | | + | | schwarz | schwarz |
| 17 | | + | | | schwarz | schwarz |
| 18 | | | + | | schwarz | schwarz |
| 19 | | | | + | schwarz | schwarz |
| 20 | | | | | schwarz | schwarz |
| 21 | | | + | | schwarz | schwarz |
| 22 | | | | | schwarz | schwarz |
| 23 | | | | + | schwarz | schwarz |
| 24 | + | | + | | rot | rot |
| 25 | | + | | | grün | grün |

Insgesamt zeigt das erfindungsgemäße Beispiel 2, dass mit dem vorliegenden Verfahren Peptide erzeugt werden können, die in den Vertiefungen des Substrats angeordnet sind. Die hierbei zur Anwendung kommende Festphasensynthese unter Verwendung von Partikeln mit dem eigentlich anzubringenden Molekül ermöglicht durch das zielgerichtete und ausschließliche Verbringen bestimmter Moleküle an den Reaktionsort ohne Weiteres die Herstellung längerer Oligomere, beispielsweise von Polymeren mit einer Länge von 15 Aminosäuren bzw. Aminosäurederivaten oder mehr. Somit eröffnet das vorgestellte Verfahren eine einfache Möglichkeit, stochastische Peptidarrays einer Dichte von bis zu 70 Millionen Spots/cm² herzustellen. Es ist dem Fachmann klar, dass das hierin vorgestellte Verfahren problemlos auf die Herstellung von Nukleotidarrays und/oder anderer Oligomerarrays übertragen werden kann und dass die hierin vorgestellten Oligomerarrays zur Ermittlung geeigneter Bindepartner in der Analytik Verwendung finden.

### Beispiel 3: Herstellung eines Peptidarrays mit Peptiden einer Länge von bis zu 9 Aminosäuren.

Ein stochastischer Peptidarray mit Peptiden einer Länge von bis zu 9 Aminosäuren wird hergestellt. Die erfolgreiche Synthese des Peptidarrays wird mittels Färben mit fluoreszenzmarkierten Antikörpern einschließlich anschließender Fluoreszenzdurchmusterung durchgeführt.

### 3.1. Funktionalisierung des Substrates mit Vertiefungen

Als Substrat für die Peptidarraysynthese wird ein Quarzglasobjektträger mit Vertiefungen und mit den Abmessungen von 20 mm x 20 mm x 0,5 mm verwendet. Die Fläche mit den Vertiefungen weist ein Pitch von 15 µm, einen Vertiefungsdurchmesser von 12 µm und eine Tiefe der Vertiefungen von 10 µm auf.

Vor der Herstellung des eigentlichen Peptidarrays wird der Objektträger mit einer Polymerschicht aus 10:90 PEGMA-co-MMA (Poly(ethylenglykol)methacrylat-co-methylmethacrylat) funktionalisiert.

### 3.2. Herstellung der Partikel

Sechs Arten von Partikeltypen mit unterschiedlichen Aminosäuren und Quantenpunktmarkierungen werden vor der Herstellung des stochastischen Peptidarrays synthetisiert. Die Aminosäuren sowie die Markierungen für die einzelnen Partikel werden in nachstehender Tabelle 1 aufgeführt.

**Tabelle 1**

| **Nr.** | **Fmoc-Aminosäure-OPfp** | **1 Buchstabencode** | **Quantenpunktmarker** |
|---|---|---|---|
| 1. | Alanin | A | 500 nm ("blau") |
| 2. | Asparaginsäure | D | 500 nm ("blau") |
| 3. | Lysin | K | 590 nm ("grün") |
| 4. | Prolin | P | 590 nm ("grün") |
| 5. | Tyrosin | Y | 590 nm ("grün") |
| 6. | Valin | V | 590 nm ("grün") |

### 3.2.1 Materialien

1. Festes Trägerpulver: Quasi-monodisperse Mikropartikel basierend auf quervernetztem PMMA mit einem Mediandurchmesser von 10 µm.
2. Polymermatrix: Styrol-Acrylcopolymer.
3. Aminosäure: Pulver der Fmoc-, OPfp-geschützten Aminosäure.
4. Lösung von Quantenpunkten (QD): 25 mg QD auf 4 ml Chloroform.
5. Dichlormethan (DCM).
6. Aceton.
7. Ethanol.

### 3.2.2 Durchführung

### a) Zubereitungsschritte

1 g des festen Trägerpulvers wird in das Becherglas (25 ml) eingebracht. 0,1 g der Polymermatrix und 0,01 g des Aminosäurepulvers werden in ein Gefäß 1 eingebracht. 50 µl QD-Lösung in Chloroform werden in das Gefäß 2 eingebracht. 2 ml DCM werden dem Gefäß 2 hinzugegeben. Es wird abgewartet bis die QDs in DCM gelöst sind. 30 ml Aceton werden in die Kalibrierungsbürette gegeben.

### b) Markierung des festen Trägers

8 ml DCM werden in das Becherglas mit dem festen Trägerpulver gegeben und mit dem Magnetrührer gerührt, bis eine homogene Dispersion erhalten wird. 2 ml der QD-Lösung aus dem Gefäß 2 werden in das Becherglas mit der festen Trägerdispersion gegeben. Unter Rühren des festen Trägers in DCM werden aus der Bürette 30 ml Aceton langsam zu der Dispersion für einen Zeitraum von 1 bis 1,5 Stunden gegeben. Anschließend wird das Rühren beendet und die Sedimentierung der festen Trägerpartikel abgewartet bzw. eine Zentrifuge benutzt. Die flüssige Phase der Dispersion wird entfernt, wobei die festen Trägerpartikel in dem Becherglas verbleiben. 15 ml Ethanol werden in die Kalibrierungsbürette gegeben und langsam in das Becherglas mit den festen Trägerpartikeln unter Rühren für einen Zeitraum von 10 bis 15 Minuten hinzugegeben. Das Rühren wird beendet und die Sedimentierung der festen Trägerpartikel wird abgewartet oder eine Zentrifuge benutzt. Die flüssige Phase der Dispersion wird, unter Beibehaltung der festen Trägerpartikel in dem Becherglas, entfernt.

### c) Waschen des festen Trägers

10 ml Aceton werden in das Becherglas mit dem festen Träger gegeben. Die Dispersion wird für 1 Minute gerührt. Nach Beenden des Rührvorgangs wird auf die Sedimentierung der festen Trägerpartikel gewartet bzw. die Zentrifuge benutzt. Die flüssige Phase der Dispersion wird, unter Beibehaltung der festen Trägerpartikel in dem Becherglas, entfernt. 10 ml Aceton werden in das Becherglas mit dem festen Träger gegeben. Die Dispersion wird für 1 Minute gerührt. Der Rührvorgang wird angehalten und auf die Sedimentierung der festen Trägerpartikel gewartet bzw. die Zentrifuge benutzt. Die flüssige Phase der Dispersion wird, unter Beibehaltung der festen Trägerpartikel in dem Becherglas, entfernt.

### d) Aminosäurepolymermatrixapplikation

4 ml DCM werden in das Gefäß 1 gegeben. Es wird abgewartet bis sich die Polymermatrix und das Aminosäurepulver gelöst haben. 4 ml der Lösung aus dem Gefäß 1 werden in das Becherglas mit dem festen Träger hinzugegeben. Es wird gerührt, bis eine homogene Dispersion erhalten wird und abgewartet bis eine homogene Masse aufgrund der Verdampfung von DCM aus der Dispersion auftritt. Der Rückstand wird für 2 Stunden zum vollständigen Trocknen stehen gelassen.

### 3.3 Peptidarraysynthese

Die Synthese des stochastischen Peptidarrays umfasst allgemein die folgenden Schritte:
- Fmoc - Entschützen der terminalen NH₂-Gruppen der Polymerketten bzw. Aminosäure(n).
- Einbringen der Partikelmischung, entsprechend der gewünschten Aminosäureablagerung je Schicht, in die Vertiefungen des mikrostrukturierten Substrats.
- Durchmustern des Substrats auf abgelagerte Partikel unter Verwendung eines Fluoreszenzscanners, um die Position der quantenpunktmarkierten Partikel zu bestimmen.
- Dekodieren des Fluoreszenzmusters in das entsprechende Aminosäuremuster.
- Extraktion und Binden der Aminosäuren.
- Entfernen der Partikel und Auswaschen der Rückstände nach dem Binden.
- Acetylieren (Blockieren) nicht-reagierter freier terminaler NH₂-Gruppen.

Die Abfolge dieser Verfahrensschritte wird neunmal wiederholt, jedes Mal für die entsprechende Aminosäureschicht. Bei jeder Aminosäureschicht werden ggf. vorhandene Seitenkettenfunktionalitäten geschützt und nach erfolgter Synthese aller Aminosäureschichten entschützt.

Die Arten an Aminosäuren für jeden Zyklus werden derart gewählt, dass die FLAG- und HA-Epitope mit ausreichender Wahrscheinlichkeit durch Zufallssynthese hergestellt werden können. Die Partikelmischungen, die für jede Peptidarrayschicht verwendet werden, werden in nachstehend aufgeführter Tabelle 2 gezeigt.

**Tabelle 2**

| **Schicht-Nr.** | **Partikel-Typ 1** | | **Partikel-Typ 2** | |
|---|---|---|---|---|
| | **Fmoc-Aminosäure-OPfp** | **Fluoreszenzmarker** | **Fmoc-Aminosäure-OPfp** | **Quantenpunktmarker** |
| 1. | Alanin (A) | 500 nm ("**blau**") | Lysin (K) | 590 nm ("**grün**") |
| 2. | Asparaginsäure (D) | 500 nm ("**blau**") | Tyrosin (Y) | 590 nm ("**grün**") |
| 3. | Asparaginsäure (D) | 500 nm ("**blau**") | Asparaginsäure (D) | 500 nm ("**blau**") |
| 4. | Asparaginsäure (D) | 500 nm ("**blau**") | Prolin (P) | 590 nm ("**grün**") |
| 5. | Asparaginsäure (D) | 500 nm ("**blau**") | Valin (V) | 590 nm ("**grün**") |
| 6. | Asparaginsäure (D) | 500 nm ("**blau**") | Lysin (K) | 590 nm ("**grün**") |
| 7. | Tyrosin (Y) | 590 nm ("**grün**") | Tyrosin (Y) | 590 nm ("**grün**") |
| 8. | Asparaginsäure (D) | 500 nm ("**blau**") | Prolin (P) | 590 nm ("**grün**") |
| 9. | Tyrosin (Y) | 590 nm ("**grün**") | Tyrosin (Y) | 590 nm ("**grün**") |

Die Herstellung des Peptidarrays wird folgendermaßen durchgeführt.

### 3.3.1 Fmoc-Entschützen der NH₂-Gruppen

Einmaliges Quellen der Polymerschicht aus 10:90 PEGMA-co-MMA in Dimethylformamid (DMF) für 5 Minuten. Einmaliges Entschützen der terminalen NH₂-Gruppen mit einer Lösung aus Piperidin (20 Vol.-%) und DMF (80 Vol.-%) für 30 Minuten. Zweimaliges Waschen des Substrats mit DMF für 5 Minuten. Zweimaliges Waschen des Substrates mit Methanol für 2 Minuten. Einmaliges Spülen des Substrats mit Dichlormethan (DCM) für 30 Sekunden. Trocknen des Substrats mit Argon.

### 3.3.2 Anfängliches Fluoreszenzscannen

Das Substrat wird unter Verwendung des Fluoreszenzscanners InnoScan1100 AL gemustert, um das Fluoreszenzmuster des leeren Substrats zu bestimmen.

### 3.3.3 Ablagerung der Partikel

Die Vertiefungen des Substrats werden mit einer Pulvermischung aus Partikeln befüllt. Nicht in den Vertiefungen vorliegende Partikel werden unter Verwendung von Druckluft entfernt. Partikelablagerung in den Vertiefungen wird unter Verwendung eines optischen Mikroskops überprüft. Im Falle einer geringen Füllrate werden die vorstehenden Schritte 3 wiederholt.

### 3.3.4 Nachweis der Partikel

Das Substrat wird unter Verwendung des Fluoreszenzscanners InnoScan1100 AL durchmustert, um das Fluoreszenzmuster der in die Vertiefungen abgelagerten Partikel zu bestimmen.

### 3.3.5 Aminosäureextraktion und Bindungsschritt

Das Substrat wird in eine Bindungskammer überführt. Die Bindungskammer muss hierbei lediglich den luftdichten Abschluss des Substrats ermöglichen und ausreichend Temperaturbeständig sein, um ein Erwärmen des Substrats zu ermöglichen. Die Kammer wird mit Argon befüllt. Extraktion und Anbinden der Aminosäuremoleküle an die terminalen NH₂-Gruppen der PEGMA-10/90-Schicht/Peptidketten werden in einem Ofen bei 90°C für einen Zeitraum von 60 Minuten durchgeführt. Anschließend werden 30 Minuten bis zum Abkühlen der Kammer abgewartet.

### 3.3.6 Entfernen der Partikel und Waschen des Substrats

Einmaliges Waschen des Substrats mit Aceton für 2 Minuten. Einmaliges Waschen des Substrats mit Aceton für 2 Minuten in einem Ultraschallbad. Einmaliges Waschen des Substrats mit Aceton für 2 Minuten. Säubern des Substrats mit Luft.

### 3.3.7 Blockierungsschritt

Einmaliges Quellen der Polymerschicht aus 10:90 PEGMA-co-MMA in DMF für 5 Minuten. Einmaliges Blockieren freier NH₂-Gruppen auf dem Substrat mit einer Lösung aus Essigsäureanhydrid (10 Vol.-%), Diisopropylethylamin (DIPEA) (20 Vol.-%) und DMF (70 Vol.-%) für 10 Minuten. Einmaliges Blockieren frei verbliebener NH₂-Gruppen auf dem Substrat mit der Lösung aus Essigsäureanhydrid (10 Vol.-%), DIPEA (20 Vol.-%) und DMF (70 Vol.-%) für 30 Minuten. Zweimaliges Waschen des Substrats mit DMF für 5 Minuten. Zweimaliges Waschen des Substrats mit Methanol für 2 Minuten. Einmaliges Spülen des Substrats mit DCM für 15 Sekunden. Trocknen des Substrats mit Argon.

### 3.3.8 Lagern des Substrats über Nacht

Das Substrat, d.h. der Quarzglasobjektträger mit den Vertiefungen, wird in einer Aufbewahrungsbox für Objektträger platziert. Die Aufbewahrungsbox wird mit Argon befüllt, mit Parafilm verschlossen und bei 4 °C im Kühlschrank gelagert.

### 3.4. Dekodieren der Fluoreszenzmuster

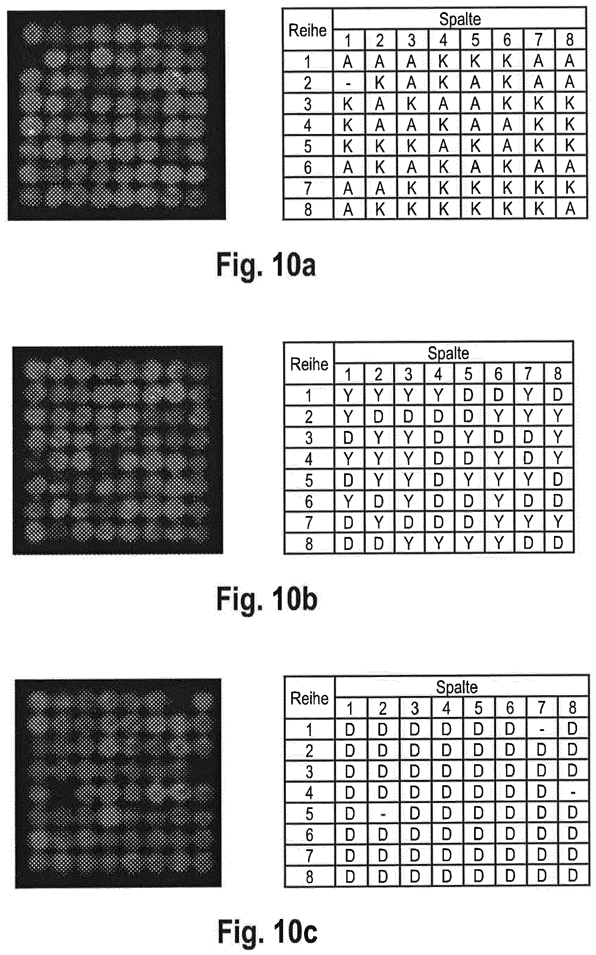

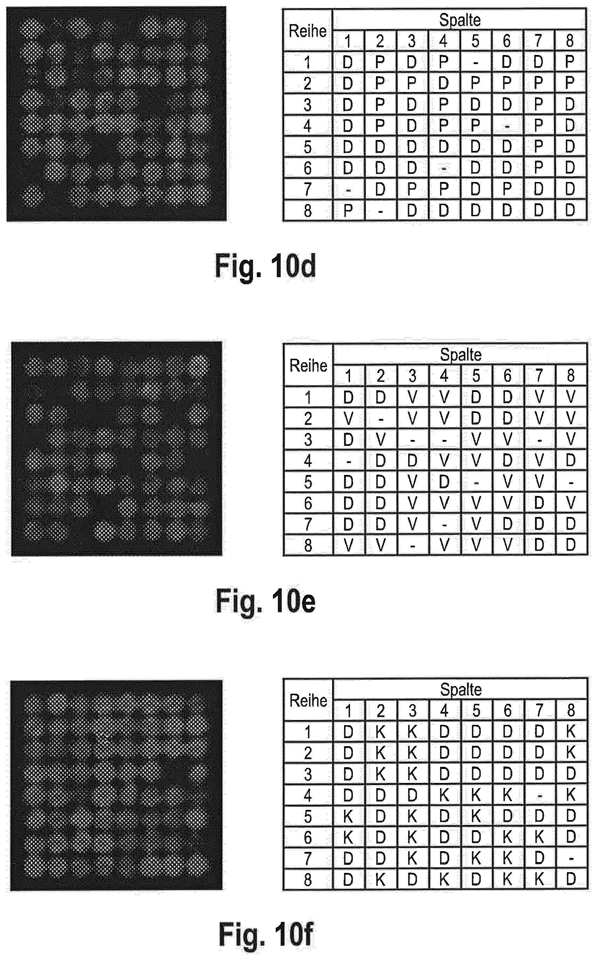

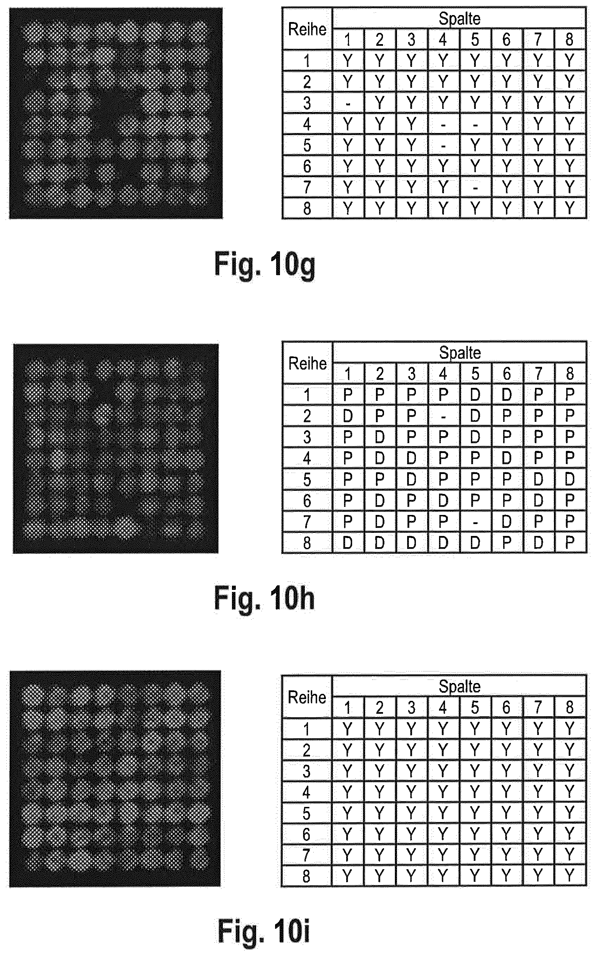

Die Fluoreszenzmuster werden für jede der neun Schichten des Peptidarrays unter Verwendung des Fluoreszenzscanners InnoScan1100 AL (Innopsys) erhalten. Anschließend werden die jeweiligen Aminosäuremuster aus dem Fluoreszenzaufnahmen dekodiert. Dies wird in den Fig. 10a bis 10e für jede der Schichten 1 bis 9 gezeigt, wobei die linke Abbildung das Fluoreszenzbild und die rechte Abbildung das entsprechende Aminosäuremuster der jeweiligen Schicht zeigt. Die Gesamtheit der Informationen der Fig. 10a bis e bildet die 3D-Ablagerungsmaske, d.h. die vollständige Information über den Syntheseort (die Koordinate der Vertiefung) eines Peptids an den Träger und die Abfolge der Aminosäuren des jeweiligen Peptids.

### 3.5. Identifizieren der Peptide in dem Array

Basierend auf den Ergebnissen des, wie in 4. beschrieben, Dekodierens der Aminosäuren werden die synthetisierten Peptidketten identifiziert. Dies wird in der nachstehend aufgeführten Tabelle 3 gezeigt.

**Tabelle 3**

| **Reihe** | **Spalte** | | | | | | | |
|---|---|---|---|---|---|---|---|---|
| | **1** | **2** | **3** | **4** | **5** | **6** | **7** | **8** |
| **1** | AYDD DDYPY | AYDPD KYPY | AYDDV KYPY | KYDPV DYPY | KDD | KDDDD DYDY | AY | ADDPV KYPY |
| **2** | | KDDP | ADDPV KYPY | KDDDV DY | ADDPD DYDY | KYDPD DYPY | AYDPV DYPY | AYDPV KYPY |
| **3** | KDDD DD | AYDPV KYDY | KYDD | ADDP | AYDDV DYDY | KDDDV DYPY | KDDP | KYDDV DYPY |
| **4** | KYDD | AYDPD DYDY | AYDDD DYDY | KDDPV K | ADDPV K | AYD | KDDPV | KY |
| **5** | KDDD DKYPY | KY | KYDDV KYDY | ADDDD D | KYDD | AYDDV DYPY | KYDPV DYDY | KDDD |
| **6** | AYDD DKYPY | KDDDD DYDY | AYDDV KYPY | KDD | ADDDV DYPY | KYDDV KYPY | ADDPD KYDY | ADDDV DYPY |
| **7** | ADD | AYDDD DYDY | KDDPV KYPY | KDDP | KDDDV K | KYDPD KYDY | KYDDD DYPY | KYDDD |
| **8** | ADDPV DYDY | KDD | KYDD | KYDDV KYDY | KYDDV DYDY | KYDDV KYPY | KDDDD KYDY | ADDDD DYPY |

| | | | | | | | | |
|---|---|---|---|---|---|---|---|---|
| *Aminosäursequenzen: vom C-Terminus (links) zum N-Terminus | | | | | | | | |

Aus der Tabelle 3 ist ersichtlich, dass das Flag-Epitop ((C-Terminus)-KDDDKYD-(N-Terminus)) und das HA-Epitop ((C-Terminus)-AYDPVDYPY-(N-Terminus)) in den Vertiefungen (Spalte 7, Reihe 8 bzw. Spalte 7, Reihe 2) stochastisch synthetisiert werden. Es ist anzumerken, dass die Peptidketten in einer bestimmten Vertiefung nicht um weitere Aminosäuren verlängert werden, falls die Vertiefung nicht mit einem Partikel in dem anschließenden Partikelablagerungsschritt befüllt wird.

### 3.6. Inkubation des Peptidarrays mit Antikörpern

Anti-HA- und Anti-FLAG-Antikörper, die mit fluoreszierenden Gruppen markiert sind, werden zum Nachweis verwendet. Der Anti-FLAG-Antikörper ist mit dem Cy5-Farbstoff (roter Kanal) konjugiert. Der Anti-HA-Antikörper ist mit dem Cy3-Farbstoff (grüner Kanal) konjugiert.

Der Inkubationsschritt wird wie nachstehend aufgeführt durchgeführt.

Vor einem Färben müssen die Fmoc- und die Seitenkettenschutzgruppen der synthetisierten Peptide entfernt werden. Zum Färben des Peptidarrays wird der Array einmal mit PBS für 15 Minuten gewaschen. Die Substratoberfläche wird einmalig mit Rocklandpuffer für 30 Minuten blockiert. Dreimaliges Waschen des Substrats mit einer Lösung aus PBS (90 Vol.-%) und Rockland-Puffer (10 Vol.-%) für 3 Minuten. Inkubieren des Peptidarrays mit einer Lösung aus PBS (2 ml), Rockland-Puffer (0,2 ml), Anti-HA-Antikörper (2 µl) und Anti-Flag-Antikörper (2 µl) für 2 Stunden. Fünfmaliges Waschen des Peptidarrays mit einer Lösung aus PBS (90 Vol.-%) und Rocklandpuffer (10 Vol.-%) für 2 Minuten. Einmaliges Spülen des Peptidarrays mit Tris-Puffer (pH ∼ 7) für 15 Sekunden.

### 3.7. Fluoreszenzdurchmusterung

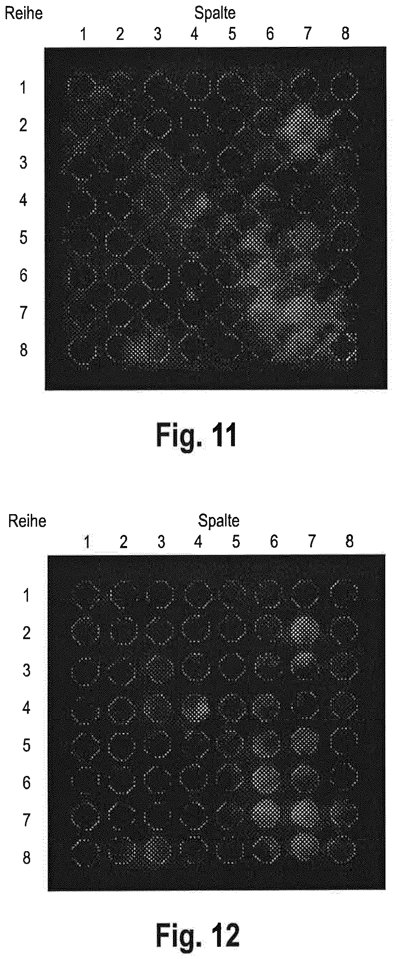

Nach der Inkubation wird der Peptidarray mit dem vorstehend beschriebenen Fluoreszenzscanner InnoScan1100 AL (Innopsys) durchmustert. Die dabei erhaltene Fluoreszenzaufnahme (Fig. 11) zeigt das Fluoreszenzmuster des inkubierten stochastischen Peptidarrays, wobei ein Koordinatensystem zur Vereinfachung der Bildanalyse angegeben ist.

### 3.8. Ergebnis

Nach erfolgter Bildverarbeitung werden die Fluoreszenzsignale den jeweiligen Vertiefungen des Substrats zugeordnet (Fig. 12). Fig. 12 zeigt das verarbeitete Bild des Fluoreszenzmusters des inkubierten stochastischen Arrays, wobei ein Koordinatensystem zur Vereinfachung der Bildanalyse bereitgestellt wird.

Wie den Fig. 11 und 12 entnommen werden kann, wird der Peptidarray unter Verwendung des erfindungsgemäßen stochastischen Verfahrensansatzes erfolgreich hergestellt. Die gewünschten Peptidketten wurden vollständig synthetisiert, wie durch erfolgreiche Fluoreszenzmarkierung zum Bestimmen der jeweiligen Fluoreszenzmarkierung gezeigt wird.

Das stärkste Signal in dem grünen Kanal (das durch den Anti-HA-Antikörper bereitgestellt wird, der mit dem CY3-Farbstoff konjugiert ist) entspricht dem stochastisch synthetisierten HA-Epitop.

Das FLAG-Epitop, ebenso wie mehrere Peptide, die das Fragment der Aminosäuresequenz *KYD**K/D* aufweisen, zeigen starke Signale in dem roten Kanal (die durch den Anti-Flag-Antikörper hervorgerufen werden, der mit dem CY5-Farbstoff konjugiert ist).

## Patentansprüche

1. Verfahren zur Herstellung eines Oligomerarrays, umfassend die Schritte:
(a) Bereitstellen eines Substrats mit einer Vielzahl von Vertiefungen;
(b) Stochastisches Einbringen eines ersten Partikels mit einem ersten Molekül in eine Vertiefung derart, dass nicht im Vorfeld bestimmt wird, welches erste Molekül an welchem Ort fixiert wird;
(c) Freisetzen des ersten Moleküls von dem ersten Partikel;
(d) Binden des ersten Moleküls an ein zweites Molekül unter Bildung eines Oligomers, wobei das zweite Molekül in der Vertiefung immobilisiert ist, wobei nach dem Schritt (b), (c) oder (d) eine Bestimmung des detektierbaren Markers in Abhängigkeit einer Position der Vertiefung auf dem Oligomerarray erfolgt;
(e) Wiederholen der Schritte (b) bis (d) unter Verlängerung des Oligomers; wobei
mindestens ein erster Partikel und/oder ein erstes Molekül einen detektierbaren Marker aufweist, wobei eine Vielzahl erster Partikel verwendet wird; und
(f) Erstellen einer 3D-Ablagerungsmaske aus den Bestimmungen des detektierbaren Markers in Abhängigkeit der Position der Vertiefung auf dem Oligomerarray, wobei die 3D-Ablagerungsmaske alle Informationen über den Ort und den Aufbau des Oligomers enthält.

2. Verfahren nach Anspruch 1, wobei in Schritt (b) das Einbringen eines einzelnen ersten Partikels mit einem ersten Molekül in eine einzelne Vertiefung erfolgt.

3. Verfahren nach einem der vorstehenden Ansprüche, wobei das erste Partikel eine Polymermatrix umfasst, in der das erste Molekül eingebettet ist.

4. Verfahren nach einem der vorstehenden Ansprüche, wobei mindestens eine Vertiefung während Schritt (c) und/oder Schritt (d) abgedichtet ist.

5. Verfahren nach einem der vorstehenden Ansprüche, wobei der detektierbare Marker ein entfernbarer, detektierbarer Marker ist, der mittels Lichtmikroskopie bestimmt werden kann.

## Claims

1. A method of producing an oligomer array, comprising the steps of:
(a) providing a substrate with a plurality of recesses;
(b) placing a first particle with a first molecule within a recess stochastically so that initially it is not determined which first molecule is fixed at which location;
(c) releasing the first molecule from the first particle;
(d) binding the first molecule to a second molecule while forming an oligomer, wherein the second molecule is immobilized within the recess, wherein after step (c) or (d) a detection of the detectible marker is done depending on a position of the recess on the oligomer;
(e) repeating steps (b) to (d) while elongating the oligomer; wherein
at least a first particle and/or a first molecule comprises a detectable marker, wherein a plurality of first particles is used; and
(f) generating a 3D deposition mask as step from the determination of the detectable marker in dependence of the position of the recess on the oligomer array, wherein the 3D deposition mask contains all the information with respect to the location and the structure of the oligomer.

2. The method of claim 1, wherein in step (b) the placing of the first particle with the first molecule is done in a single recess.

3. The method of any of the preceding claims, wherein the first particle comprises a polymer matrix within which the first molecule is embedded.

4. The method of any of the preceding claims, wherein at least one recess is sealed during step (c) and/or step (d).

5. The method of any of the preceding claims, wherein the detectable marker is a removable, detectable marker that can be detected by means of light microscopy.

## Revendications

1. Procédé pour la préparation d'un réseau d'oligomères, comprenant les étapes :
(a) mise à disposition d'un substrat doté d'une pluralité d'évidements ;
(b) introduction stochastique d'une première particule dotée d'une première molécule dans un évidement de telle sorte qu'il n'est pas déterminé au préalable quelle première molécule sera fixée à quel endroit ;
(c) libération de la première molécule de la première particule ;
(d) liaison de la première molécule à une deuxième molécule avec formation d'un oligomère, la deuxième molécule étant immobilisée dans l'évidement, une détermination du marqueur détectable en fonction d'une position de l'évidement sur le réseau d'oligomères étant réalisée après l'étape (b), (c) ou (d) ;
(e) répétition des étapes (b) à (d) avec allongement de l'oligomère,
au moins une première particule et/ou une première molécule présentant un marqueur détectable, une pluralité de premières particules étant utilisée ; et
(f) établissement d'un masque d'accumulation 3D à partir des déterminations du marqueur détectable en fonction de la position de l'évidement sur le réseau d'oligomères, le masque d'accumulation 3D contenant toutes les informations sur l'emplacement et la construction de l'oligomère.

2. Procédé selon la revendication 1, où dans l'étape (b) l'introduction d'une première particule individuelle dotée d'une première molécule est réalisée dans un évidement individuel.

3. Procédé selon l'une quelconque des revendications précédentes, la première particule comprenant une matrice polymère, dans laquelle la première molécule est incorporée.

4. Procédé selon l'une quelconque des revendications précédentes, au moins un évidement étant scellé pendant l'étape (c) et/ou l'étape (d).

5. Procédé selon l'une quelconque des revendications précédentes, le marqueur détectable étant un marqueur détectable, éliminable, qui peut être déterminé au moyen de microscopie optique.
